# EUROPEAN PATENT APPLICATION

(11) **EP 3 470 507 A1**
(43) Date of publication of application: **17.04.2019**
(21) Application number: 18209880.6
(22) Date of filing: 02.09.2016
(51) Int. Cl.: C11D 9/04, C11D 9/02, A61K 8/97, A61Q 5/02, A61Q 19/10, C11D 11/00, C11D 9/38, C11D 13/00, C11D 13/02, A61K 8/9789, A61K 8/9794

(54) **HERBAL EXTRACTION AND MANUFACTURING PROCESS**

(30) Priority: 03.05.2016 TR 201605777
(62) Divisional of application: 16784296.2
(71) Applicant: Bioarge Bitkisel Kozmetik Arastirma Gelistirme Muhendislik Ltd. STI., Istanbul (TR)
(72) Inventor: OZAYMAN, NURI MURAT, 34197 Istanbul (TR)
(74) Representative: Coral, Nükhet Serra Yardimci

(57) **Abstract**

An improved soap composition and methods of manufacture thereof are disclosed. In one embodiment, a soap composition is produced from a saponification reaction between: a first mixture comprising an oil and a vinegar component, the vinegar component including an extraction medium of natural vinegar and extract from a natural plant additive; and a second mixture comprising an alkaline solution. In another embodiment, the invention relates to the use of soap raw materials as the extraction medium of herbal ingredients for use in soap, cosmetic, and/or household products. The invention eliminates extra steps needed to separate the extract from a solvent as the extraction medium itself is one or more of the raw materials of the product. In yet another embodiment, the invention relates to increasing the zeta-potential of relevant insoluble particles in the solution of a soap base by the incorporation of zeta-potential increasing component(s).

## Description

### TECHNICAL FIELD

This invention relates to an improved natural soap base composition and/or other composition for use in cosmetic, personal care, and/or household products, and manufacturing methods thereof.

### BACKGROUND

Natural soap has been the safest cleaning product for centuries. "Natural" soap is generally a derivative of vegetable oils or animal fats mixed with an alkaline solution, which are each renewable natural raw materials (in other words, existing in nature). When triglycerides (including fatty acids, glycerol molecules, and aliphatic chains) of the oil (or mixture of oils) or fat (or mixture of fats) mix with an alkaline solution, they undergo a chemical process/reaction of "saponification" to produce soap. Akin to mixing an acid with a base, a saponification reaction may include a hydroxide (OH⁻) attacking a carboxyl group of the fatty acid which is attached to a glycerol. This causes the carboxyl to break away and form carboxylic acid. The alkaline base (e.g., lye, potassium hydroxide, and/or sodium hydroxide) is attracted to the carboxylic acid and forms a salt with the aliphatic chain hanging off the side, thereby forming a single molecule of "soap". This saponification chemical reaction may continue until all the base or fatty acids are spent. Thus, a natural soap does not include synthetic chemical ingredients and/or process reactants (in other words, ingredients or reactants synthesized by man).

Natural soaps and natural soap-based personal care products may not have a large demand because of some shortcomings. Natural soaps and natural soap-based products can have, although not limited to, the following disadvantages.
1) A traditional natural soap is a sodium or potassium salt of a fatty acid, or a combination of fatty acids. This is why natural soap may be vulnerable to the attack of water hardness, which is mainly calcium and magnesium ions present in water. Calcium and magnesium ions react with the sodium or potassium salts of the fatty acids to form their calcium and magnesium salts, which are not water soluble, and therefore the soap is rendered ineffective in hard water.
2) The formed insoluble calcium and magnesium salts cause an unwanted residue (also referred to as "soap scum") in the bath tub or on other surfaces of the bathing/showering area.
3) Traditional natural soaps inherently and readily dry out in open atmosphere.
4) Traditional natural soaps have a drying effect on the skin.
5) Traditional natural soaps have a high pH value, as they are typically alkaline. For easy saponification, the industry may traditionally use excess lye, sodium hydroxides, or potassium hydroxides for saponification reactions.

Previously, synthetic chemicals have been used to eliminate or reduce such shortcomings of natural soaps. However, soaps modified with synthetic chemicals can no longer be classified as "natural" (having or using components existing in or derived from nature) and may have negative impacts on human health and/or ecosystems. More frequently, synthetic surface active materials are deployed in so called "liquid soaps", cosmetics, and personal care and household products. As modern consumers have an increasing demand for natural products, detergent producers may advertise that many hand soaps, shampoos, and other personal care products contain some beneficial natural extract or additive. However, synthetic ingredients have previously formed the basis for the soap base, surface active materials, and/or many other functional groups, such as microbial preservatives, antioxidants, and humectants, and therefore cannot be considered fully "natural" products.

Natural soap cleans the skin by removing the oil and dirt from the skin and keeping them in solution in micelles that will not again adhere to the skin. In contrast, detergents with synthetic chemical ingredients, including synthetic liquid soap, will compete with the oil on the skin to adhere to the skin. This synthetic chemical film is harder to rinse off from the skin and consumes more water. Further, such synthetic surface active chemicals enter much deeper into the skin, which removes the oil that is necessary for maintaining skin moisture and causes dryness.

In addition, if we consider the thousands of tons of synthetic detergents drained to our underwater reserves every day, and the environmental laws employed to regulate such synthetics discharge, acceptable natural soap bases are highly desirable from both an ecological and business perspective.

Accordingly, soaps and soap-based personal care products are needed which are healthier and safer to use, desirable for ecological health, and eliminate or reduce the shortcomings of a natural soap base to acceptable levels by natural means and methods.

Furthermore, soaps, cleaning products, cosmetic products and personal care products (hereafter referred to as "products") include more and more herbs, in terms of variety and amount, as the demand from the conscious consumer increases.

However, several problems have been encountered during herbal extraction processes, as described below.
1) Chemical solvents that are used for an extraction process may remain in the extract and cause contamination even in small amounts. Solvents, such as carbon dioxide, may react with the basic components due to their acidic nature and remain in the extract as more stable compounds but nonetheless cause contamination. This contamination may cause complications in situations where the herbal remnants are desired for subsequent use, such as for animal feed.
2) Organic extracts that are stored for long periods of time have a higher risk of deterioration and becoming "stale". Extracts that are produced in necessary amounts in the production area and then used are more assured to remain fresh and potent.
3) Extraction costs are increased by the need to devote capital funds for the foundation of facilities or equipment for the means of extraction, such as for super critical carbon dioxide use and operational costs such as for separation of the desired extract from the solvent.

Accordingly, there is a need for methods that provide for the preparation of desired natural extracts, and which eliminate or reduce to acceptable levels the shortcomings of prior herbal extraction means and methods as noted above.

### SUMMARY

The present invention advantageously and surprisingly eliminates or reduces the above-mentioned shortcomings of prior natural soaps by including a natural additive, a vinegar component. The vinegar component may be added to an oil reactant prior to a saponification reaction with an alkaline solution, in combination with other elements or process steps. Alternatively, a neutralized vinegar component or an herbal extract vinegar infusion component may be added to a soap base after a saponification reaction. Thus, a soap composition is prepared with a vinegar component(s) and/or herbal extract infusion(s).

Advantageous manufacturing methods are also provided for herbal extraction that advantageously eliminate or reduce the above-mentioned shortcomings of prior herbal extraction means and methods. In particular, manufacturing methods of herbal extraction are provided that utilize a raw material ingredient of the end product as an extraction medium for the desired herbal extract. In one example, methods are provided for using a soap raw material as an extraction medium for soluble components of an herb of interest.

In one embodiment, a soap base composition is produced from a saponification reaction between: (a) a first mixture including a first herbal extract infusion having soluble components of an herb solvated in a first extraction medium, wherein the first extraction medium is a first saponification reactant for producing the soap base composition; and (b) a second mixture including a second saponification reactant for producing the soap base composition.

The soap base composition may have the following alternative components, which may also be combined in various applicable and functioning combinations within the scope of the present invention: the first herbal extract infusion includes a first extraction medium selected from the group consisting of vinegar, turpentine, oil, fat, milk, sodium hydroxide, potassium hydroxide, glycerin, water, and a combination thereof; the second mixture includes a second herbal extract infusion having soluble components of an herb solvated in a second extraction medium, wherein the second extraction medium has a same composition as the second saponification reactant; the herb solvated in the second extraction medium is an herbal residue solvated in the first extraction medium, the herb solvated in the first extraction medium undergoing multiple extractions in different extraction media; the herb solvated in the first extraction medium undergoing multiple extractions in different extraction media is selected from the group consisting of sunflower seeds, almonds, hazelnuts, corn, olive and olive kernels, and palm and palm kernels; the second saponification reactant is an alkaline hydroxide including one of a sodium hydroxide, a potassium hydroxide, and a combination thereof; the second mixture includes potassium hydroxide between 11 to 14 wt%, sodium hydroxide between 4 to 5 wt%, and water between 37 to 44 wt%; the soap base composition has a pH value between about 9 and about 10.5; the saponification reaction takes place at a temperature between 70 degrees Celsius and 99 degrees Celsius; and a combination thereof.

In yet another embodiment, a soap base composition is produced from a saponification reaction between: (a) a first mixture including a first herbal extract infusion having soluble components of a first herb solvated in a first extraction medium, wherein the first extraction medium is a first saponification reactant for producing the soap base composition, the first extraction medium selected from the group consisting of vinegar, turpentine, oil, fat, milk, glycerin, water, and a combination thereof; and (b) a second mixture including a second herbal extract infusion having soluble components of a second herb solvated in a second extraction medium, wherein the second extraction medium is a second saponification reactant for producing the soap base composition, the second extraction medium selected from the group consisting of sodium hydroxide, potassium hydroxide, and a combination thereof.

In yet another embodiment, a process for preparing a soap base composition is provided, the process comprising: extracting soluble components of an herb with a first extraction medium to provide a first herbal extract infusion, wherein the first extraction medium is the same compound as a first saponification reactant for producing the soap base composition; providing a first mixture including the first herbal extract infusion and the first saponification reactant; providing a second mixture including a second saponification reactant; and mixing the first mixture with the second mixture to provide a saponification reaction producing the soap base composition.

In yet another embodiment, a process for preparing a soap base composition is provided, the process comprising: extracting soluble components of a first herb with a first extraction medium to provide a first herbal extract infusion, wherein the first extraction medium is a first saponification reactant for producing the soap base composition; extracting soluble components of a second herb with a second extraction medium to provide a second herbal extract infusion, wherein the second extraction medium is a second saponification reactant for producing the soap base composition, and wherein the second herb extracted in the second extraction medium is the same as the first herb extracted with the first extraction medium, the first herb extracted with the first extraction medium undergoing multiple extractions in different extraction media; and mixing the first herbal extract infusion with the second herbal extract infusion at a temperature between about 70 degrees Celsius and about 99 degrees Celsius to provide a saponification reaction producing the soap base composition having a pH value between about 9 and about 10.5.

In yet another embodiment, an herbal extraction method for natural products is provided, the method comprising: extracting soluble components of a first herb with a first extraction medium to provide a first herbal extract infusion, wherein the first extraction medium is a first raw material for producing a cosmetic product; extracting soluble components of a second herb with a second extraction medium to provide a second herbal extract infusion, wherein the second extraction medium is a second raw material for producing the cosmetic product; and mixing the first herbal extract infusion with the second herbal extract infusion to produce the cosmetic product.

### DESCRIPTION OF THE DRAWINGS

These and other features, aspects, and advantages of the present invention will become better understood when the following detailed description is read with reference to the accompanying drawings in which like characters represent like parts throughout the drawings. Unless noted, the drawings may not be drawn to scale.
FIG. 1 is a flowchart illustrating a method of manufacturing a natural soap with a vinegar component in accordance with an embodiment of the present invention.
FIG. 2 is a flowchart illustrating a method of manufacturing a natural soap with a vinegar component in accordance with another embodiment of the present invention.
FIG. 3 is a flowchart illustrating a method of manufacturing a natural soap with a vinegar component in accordance with yet another embodiment of the present invention.
FIG. 4 is a flowchart illustrating a method of manufacturing a natural soap with a vinegar component in accordance with yet another embodiment of the present invention.
FIG. 5 is a flowchart illustrating a method of manufacturing a natural soap with a vinegar component in accordance with yet another embodiment of the present invention.
FIG. 6 is a flowchart of a method of herbal extraction in accordance with an embodiment of the present invention.

### DETAILED DESCRIPTION

The present invention advantageously and surprisingly eliminates or reduces the above-mentioned shortcomings of prior natural soaps by including a natural additive, a vinegar component. The vinegar component may be added to an oil reactant prior to a saponification reaction with an alkaline solution, in combination with other elements or process steps. Alternatively, a vinegar component may be added to a soap base after a saponification reaction as a neutralized vinegar component or as an herbal extract vinegar infusion component. Thus, a soap composition is prepared with a vinegar component(s) as a vinegar solution and/or a vinegar infusion of an herbal extract(s).

Advantageous manufacturing methods are also provided for herbal extraction that advantageously eliminate or reduce the above-mentioned shortcomings of prior herbal extraction means and methods. In particular, manufacturing methods of herbal extraction are provided that utilize a raw material ingredient of the end product, such as a soap or cosmetic composition, as an extraction medium for the desired herbal extract.

### Soap Composition Including A Vinegar Component

Soap base compositions in accordance with the present invention, its products, and production process steps, substantially eliminate or reduce the disadvantages of the prior traditional natural soaps described above. In one embodiment, a soap base composition is provided, formed from a saponification reaction between (a) a first mixture comprising an oil and a vinegar component, the vinegar component including an extraction medium of natural vinegar and extract from a natural plant additive, and (b) a second mixture including an alkaline solution. In one example, between 5-30 wt% natural vinegar (e.g., vinegar including about 4.5 wt% acetic acid) is mixed with pre-saponified oil (or a mixture of pre-saponified oils) (in other words, the vinegar is between 5% to 30% by weight of the oil composition). In another example, between 2-35 wt% natural vinegar is mixed with pre-saponified oil (in other words, the vinegar is between 2% to 35% by weight of the oil composition). A pre-saponified component (e.g., pre-saponified oil) is a component that has not undergone a saponification reaction (but may do so in the future). The oil and vinegar mixture is then mixed with an alkaline solution to undergo a saponification reaction to produce a natural soap base. The oil and vinegar component reacts with the alkaline solution in a saponification process. Surprisingly and advantageously, the addition of a vinegar component to oil, prior to saponification, removes many of the disadvantages of natural soap base and liquid natural soap described above, including but not limited to making the produced soap base effective in hard water, effective in reducing soap scum, and effective in limiting odor.

The vinegar component that may be used in the present invention is a dilute solution of acetic acid, which can further include aromatic and coloring matters, protein matters, and inorganic substances. In one example, natural vinegar can be prepared by acetic fermentation of alcoholic beverages, whereby a natural biological product is obtained, in one example obtaining an aqueous solution including 3-20 wt% of acetic acid. The transformation of alcoholic beverages into vinegar, in one example, is achieved by oxidizing the ethyl alcohol by means of the enzyme alcohol oxidase produced by aerobic bacteria of acetobacter species. Natural vinegar may additionally include 10 inorganic salts, in particular those of zinc, potassium, lithium, and sodium; sugars, in particular glucose, fructose, and xylitol; ethanol; and vitamins (e.g., vitamins B and D). The vinegar may be in the form of a clear solution whose color varies from orange-yellow to red.

Alcoholic solutions, such was wine, beer, or the like can be used for fermentation. Natural vinegar can also be obtained from sugar solutions derived from apples, plums, currant, pears, and other fruits by fermentation in the presence of the appropriate bacteria, whereby sugars are converted initially into alcohol and then into acetic acid. For example, the vinegar can be one of apple vinegar, rice vinegar, wine vinegar, cider vinegar, lemon vinegar, blackberry vinegar, raspberry vinegar, sugar cane vinegar, sugar beet vinegar, and a combination thereof.

The vinegar component may also include a vinegar infusion of an herbal extract, in which vinegar is advantageously employed as an extracting solvent medium ("extraction medium") and/or carrier for the addition of enhancing and functionally-complementary herbs or plant parts. Such a vinegar "infusion" of one or more herbs or plant parts include soluble components of the one or more herbs or plant parts in vinegar and enhance the properties or functionality of vinegar prior to or during the saponification reaction to produce the soap base composition. In one example, a vinegar infusion of one or more herbs or plant parts is prepared by soaking or steeping the plant part in a vinegar solution for a time period (e.g., on the order of hours, days or weeks), and then filtering any remaining solids from the mixture to separate the liquid part including vinegar soluble components of the plant. The separated liquid part is termed the infusion of the herb or plant (e.g., a vinegar infusion of the herb or plant).

In another embodiment, a soap base composition is provided, produced from a reaction between: (a) a first mixture including a plurality of unsaponified oils and a vinegar infusion of natural vinegar and a natural plant additive; and (b) a second mixture including an alkaline solution, having one of a sodium hydroxide, a potassium hydroxide, and any combination thereof. The reaction is heated to a temperature between 70 degrees Celsius and 95 degrees Celsius, the produced soap base composition has a pH value between about 9.0 and about 10.5, and the vinegar infusion is between 5 % to 30 % by weight of the oil composition.

In the above embodiments, the following alternatives may be combined in various applicable and functioning combinations within the scope of the present invention. In one alternative, the oil used as a reactant can be one selected from the group consisting of olive oil, sunflower seed oil, corn oil, coconut oil, palm oil, palm kernel oil, castor oil, and any combination thereof. In another alternative, the natural vinegar can be selected from the group consisting of apple vinegar, rice vinegar, wine vinegar, cider vinegar, lemon vinegar, sugar beet vinegar, sugar cane vinegar, blackberry vinegar, raspberry vinegar, and any combination thereof. In another alternative, the natural vinegar can be between 5 % to 30 % by weight of the first oil mixture. The natural vinegar can have an acetic acid concentration between 2 wt % and 8 wt %, in one example. The natural vinegar can have an acetic acid concentration between 2 wt % and 30 wt %, in another example.

In another alternative, the natural plant additive is selected from the group consisting of coriander, thyme, clove, rosemary, aloe vera, comfrey, colt's foot, nettle, rosewood, chamomile, tragacanth, locust bean, soy bean, fumitory, and any combination thereof. In one example, the natural plant additive is coriander that enhances a soap property of being effective in hard water. In another example, the natural plant additive is one of thyme, clove, rosemary, and any combination thereof, that enhances a soap property of resistance to bacterial infection. In another example, the natural plant additive is one of aloe vera, comfrey, colt's foot, and any combination thereof, that enhances a soap property of moisturizing skin. In another example, the natural plant additive is one of stinging nettle, rosewood, chamomile, and any combination thereof, that enhances a soap anti-allergic property. In another example, the natural plant additive is one of lemon, orange, and any combination thereof, that enhances milder soap pH level. In another example, the natural plant additive is one of tragacanth, locust bean, and any combination thereof, that protects against atmospheric drying of the soap composition.

In another alternative, the second mixture includes an alkaline hydroxide including one of a sodium hydroxide, a potassium hydroxide, and a combination thereof.

The present invention eliminates or at least reduces many of the above and below described shortcomings of natural soaps and products based on them, advantageously utilizing natural products and processing steps without the addition of synthetic chemicals.

### Effectiveness Against Hard Water

A central disadvantage of natural soaps, until the present invention, has been their ineffectiveness in hard water. By adding the present vinegar additive to pre-saponified oil to form a first mixture, and then mixing with a second mixture including an alkaline solution, a saponification reaction takes place to produce a natural soap product of the present invention which is effective in hard water. The acetic acid of the vinegar reacts with a part of the lye to form sodium acetate. The formed sodium acetate reacts with soluble calcium and magnesium ions of the hard water to form their water soluble acetates. Thus, the calcium and magnesium ions are bound into soluble acetates, no longer attacking the sodium or potassium salts of the fatty acids. Accordingly, the soap produced after the saponification reaction is rendered effective in hard water.

Table 1 below provides results from experiments conducted to show effectiveness in hard water of a soap produced with the present vinegar additive. 100 ml of 1 wt%, 2 wt%, and 5 wt% solutions of liquid soap with and without vinegar were prepared using tap water. The solutions of liquid soap including vinegar were prepared with 2 wt% vinegar of the liquid soap, added prior to saponification. The solutions were thoroughly shaken at an angle of 45 degrees in a 500 ml graded cylinder and then the heights of formed foams were measured at 0 minutes, 0.5 minutes, and 5 minutes. The heights of the foam formed after shaking were determined to be proportional to soap effectiveness as the formation of calcium and magnesium salts decrease foam production. In the 1% soap solutions comparison with no vinegar and vinegar, an increase in the height of soap foam was generally observed in the solutions including vinegar. For example, the 1% solution of soap with vinegar yielded about a 235% larger volume of foam in 0 minute and 0.5 minutes after shaking and about a 260% larger volume of foam in 5 minutes after shaking as compared to the 1% solution of soap without vinegar. This is a clear indication of vinegar rendering soap solutions effective in hard water.

**TABLE 1**

| **TAP WATER SOLUTION OF LIQUID SOAP WITH NO VINEGAR** | | | | | | | | | |
|---|---|---|---|---|---|---|---|---|---|
| | **1% SOAP SOLUTION** | | | **2% SOAP SOLUTION** | | | **5% SOAP SOLUTION** | | |
| Foam height | **0.min** | **0.5min** | **5min** | **0.min** | **0.5min** | **5min** | **0.min** | **0.5min** | **5min** |
| (mm) | 155 | 155 | 115 | 450 | 450 | 150 | 365 | 350 | 300 |
| | | | | | | | | | |

| **TAP WATER SOLUTION OF LIQUID SOAP WITH 2% VINEGAR** | | | | | | | | | |
|---|---|---|---|---|---|---|---|---|---|
| | **1% SOAP SOLUTION** | | | **2% SOAP SOLUTION** | | | **5% SOAP SOLUTION** | | |
| Foam height | **0.min** | **0.5min** | **5min** | **0.min** | **0.5min** | **5min** | **0.min** | **0.5min** | **5min** |
| (mm) | 365 | 365 | 300 | 430 | 425 | 420 | 450 | 440 | 300 |

The inventor has further discovered that this property of vinegar to make natural soap effective in hard water, is enhanced by vinegar infusion of coriander or soy bean prior to saponification (or alternatively after saponification in some formulations).

### Effectiveness Against Soap Scum (Zeta Potential)

Another disadvantage of natural soaps, prior to the present invention, has been the formation of soap scum. The present vinegar additive minimizes or reduces the formation of significant quantities of insoluble salts of the soap fatty acids and thereby minimizes or reduces the risk of soap scum formation.

The inventor has discovered that solutions of the present invention soap base in water has a significantly higher zeta potential. It has been observed that the addition of vinegar to an oil component prior to or after saponification significantly increases the zeta potential of the produced soap base in solution. As a result of higher zeta potential levels, insoluble soap scum will tend to stay in emulsion and not settle onto a surface as scum.

Settling of soap scum and unwanted insolubles is a function of the zeta potential of such dispersions. According to the DLVO (Derjaguin, Landau, Verwey and Overbeek) theory, the total electronic charge of insoluble particles determines the stability of the emulsion. Absolute values less than ±50 mV indicate an unstable range.

Experiments were conducted to determine the effectiveness against soap scum of a soap produced with the present vinegar additive. Samples to be compared were placed in disposable cartridges and a minimum of three charge measurements per sample were conducted on Malvern Instruments, Malvern Nano ZSP. A 0.5 wt% solution of liquid soap was prepared. The zeta potential values were measured and an average value of -56 mV was determined for liquid soap with no additives in tap water. -56 mV is a critical value close to the precipitation point of the insoluble particles in emulsion. The results showed that the addition of pH adjusted vinegar to soap significantly increased the zeta potential of a natural soap emulsion; thus, effectively decreasing the probability of insolubles settling to produce soap scum. Water solution of vinegar additive was brought to the pH of the soap before addition to the liquid soap, and then added to 10% of the total weight of the initial fatty acids.

Similar success was achieved with the addition of Guar Gum, Tara Gum, CMC, Sodium Acetate, Borax, PVP, Mono Glycerate, and Sodium Citrate, as well. The following additives in Table 2.1 below were added in quantities equal to 10% of the total weight of the initial fatty acids. Water solutions of the following additives were brought to the pH of the soap before addition to the liquid soap.

**Table 2.1**

| Additive | Zeta Potential (mV) |
|---|---|
| None | -56 |
| Guar Gum | -60 |
| Tara Gum | -61 |
| CMC | -61 |
| Sodium Acetate | -61 |
| Borax | -62 |
| Vinegar | -63 |
| PVP | -66 |
| Mono Glycerate | -66 |
| Sodium Citrate | -71 |

When producing soap from oils instead of fatty acids, some superfat (adding lye less than the stoichiometric amount) yields a small amount of unsaponified monoglycerides of the existing fatty acids that have been surprisingly discovered to be functioning as strong zeta potential enhancers.

Table 2.2 below provides results from further experiments conducted to show the effectiveness in preventing soap scum by a soap produced with the present vinegar additive. 100 ml of 1 wt%, 2 wt%, and 5 wt% solutions of liquid soap with and without vinegar were prepared using tap water. Values for solution pH and conductivity were determined. Also, a laser beam was passed through each beaker of solution and the visibility of the beams was observed and rated as a function of the quantity of insolubles in suspension. A 1+ rating refers to trace amounts of insolubles, the least amount on this scale. A 2+ rating refers to an acceptably small amount of insolubles, or a higher amount of insolubles than a 1+ rating. A 3+ rating refers to a more visible amount of insolubles, or a higher amount of insolubles than a 2+ rating. A 4+ rating refers to the highest visible amount of insolubles, or a higher amount of insolubles than a 3+ rating. The intensity of the visible beam is an indication of the quantity of insolubles that have a potential to settle as scum, whereas the zeta potential of insolubles is an indicator of their tendency to settle.

The solution samples of soap with vinegar had lower intensity ratings of 2+ compared to the solution samples of soap without vinegar, which had higher intensity ratings of 4+. The addition of vinegar not only lowered the quantity of insolubles that were formed but also increased the zeta potential of the soap emulsion, thereby decreasing the tendency of formed insolubles to settle as soap scum.

**TABLE 2.2**

| **PH, CONDUCTIVITY, and INTENSITY OF OBSERVABLE INSOLUBLES IN SUSPENSION** | | | |
|---|---|---|---|
| 1+ (trace amounts) | 2+ (acceptably small amount) | | |
| 3+ (more visible amount) | 4+ (highest visible amount) | | |

| **SOLUTION SAMPLE** | **PH** | **CONDUCTIVITY** | **INTENSITY** |
|---|---|---|---|
| SOAP-NO VINEGAR + TAP WATER 1% | 9,364 | 884 | 4+ |
| SOAP-NO VINEGAR + TAP WATER 2% | 9,549 | 1089 | 4+ |
| SOAP-NO VINEGAR + TAP WATER 5% | 9,538 | 1620 | 4+ |
| SOAP-5% VINEGAR + TAP WATER 1% | 8,922 | 870 | 2+ |
| SOAP-5% VINEGAR + TAP WATER 2% | 9,255 | 1156 | 2+ |
| SOAP-5% VINEGAR + TAP WATER 5% | 9,255 | 1635 | 2+ |

The inventor has further discovered that this property of vinegar to make natural soap effective against soap scum, is enhanced by a vinegar infusion of fumitory, an herb rich in fumaric acid, prior to saponification (or alternatively after saponification in some formulations).

### Effectiveness Against Drying

Another disadvantage of natural soaps, prior to the present invention, has been atmospheric drying of the soap causing unwanted flaking and separation of the soap product. Vinegar is an effective anti-desiccant, making the soap less vulnerable to atmospheric drying.

Table 3 below provides results from experiments which were conducted to determine the effectiveness in preventing atmospheric drying of a soap produced with the present vinegar additive. Liquid soap of the present invention formed with a vinegar additive, and a control liquid soap containing no vinegar, were left to dry for 4 weeks in open beakers. The liquid soap including vinegar had a water loss of approximately 46% as compared to the liquid soap without vinegar, which had a water low of approximately 64%. The comparison of evaporated water quantity from both samples indicated that vinegar slows down the drying process of liquid soap.

**TABLE 3**

| **ATMOSPHERIC DRYING TEST** | | | | |
|---|---|---|---|---|
| DATE | LIQUID SOAP-NO VINEGAR | | LIQUID SOAP- 5% VINEGAR | |
| | WEIGHT OF SOAP (g) | 50.0641 | WEIGHT OF SOAP (g) | 50.0269 |
| | WEIGHT OF SOAP+BEAKER (g) | 107.4835 | WEIGHT OF SOAP+BEAKER (g) | 109.3232 |
| 7/4/14 | SOAP+BEAKER | 94.8360 | SOAP+BEAKER | 100.8996 |
| 7/7/14 | SOAP+BEAKER | 91.6937 | SOAP+BEAKER | 99.1178 |
| 7/8/14 | SOAP+BEAKER | 90.5548 | SOAP+BEAKER | 98.4640 |
| 7/9/14 | SOAP+BEAKER | 89.4393 | SOAP+BEAKER | 97.8320 |
| 7/10/14 | SOAP+BEAKER | 88.1518 | SOAP+BEAKER | 97.1435 |
| 7/11/14 | SOAP+BEAKER | 87.0156 | SOAP+BEAKER | 96.4526 |
| 7/14/14 | SOAP+BEAKER | 83.8936 | SOAP+BEAKER | 94.5642 |
| 7/15/14 | SOAP+BEAKER | 83.0583 | SOAP+BEAKER | 94.0290 |
| 7/16/14 | SOAP+BEAKER | 82.3426 | SOAP+BEAKER | 93.5000 |
| 7/17/14 | SOAP+BEAKER | 81.4073 | SOAP+BEAKER | 92.8580 |
| 7/18/14 | SOAP+BEAKER | 80.5330 | SOAP+BEAKER | 92.3024 |
| 7/21/14 | SOAP+BEAKER | 78.6745 | SOAP+BEAKER | 90.7938 |
| 7/22/14 | SOAP+BEAKER | 78.1625 | SOAP+BEAKER | 90.3057 |
| 7/23/14 | SOAP+BEAKER | 77.6843 | SOAP+BEAKER | 89.7625 |
| 7/24/14 | SOAP+BEAKER | 77.3793 | SOAP+BEAKER | 89.3980 |
| 7/25/14 | SOAP+BEAKER | 76.9742 | SOAP+BEAKER | 88.9534 |
| 7/31/14 | SOAP+BEAKER | 75.7210 | SOAP+BEAKER | 86.6637 |
| 8/1/14 | SOAP+BEAKER | 75.4779 | SOAP+BEAKER | 86.2207 |
| | LOSS BY EVAPORATION % | **63.92924** | LOSS BY EVAPORATION % | **46.18016** |

The inventor has further discovered that this property of vinegar to reduce atmospheric drying of natural soap is enhanced by vinegar infusion of tragacanth or locust bean prior to saponification (or alternatively after saponification in some formulations).

### Effectiveness Against Drying Skin

Another disadvantage of natural soaps, prior to the present invention, has been the drying effect on skin, which can be caused by soaps of very high pH levels. The present invention solves the problem of skin dryness by the addition of vinegar, a very effective natural humectant.

Table 4 below shows the results from an experiment that was conducted on human volunteers' hands to show the effectiveness against skin drying of a soap produced with the vinegar additive of the present invention. The volunteers were asked to wash their hands with about 1.5 g of liquid soaps with and without vinegar, and then the moisture levels of the skin of their hands were measured at 0 minutes, 5 minutes, and 60 minutes using Courage-khazaka MPA 5, Cornometer. Initially, the volunteers were asked to wash their hands with a liquid control soap that lacks vinegar. The subjects were then asked to wash their hands with a liquid soap based on the soap base of the present invention including 5 wt% vinegar.

After washing with the control liquid soap without vinegar, moisture levels per subject consistently decreased after 5 minutes and 1 hour. However, after washing with the liquid soap including 5 wt% vinegar, moisture levels per subject consistently increased after 1 hour. The comparison of the results clearly indicates that the addition of 5 wt% vinegar to the liquid soap made from the soap base of this invention significantly increased moisture levels of the skin in all volunteers.

**TABLE 4**

| | NO VINEGAR | | | |
|---|---|---|---|---|
| SUBJECT | INITIAL MEASUREMENT | AFTER 5 MIN | AFTER 1 HOUR | DATE |
| 1001 | 35.03 | 34.83 | 33.17 | 7/4/14 |
| 1002 | 43.8 | 39.13 | 9.97 | 7/4/14 |
| 1003 | 40.67 | 37.1 | 36.1 | 7/4/14 |
| 1004 | 51.8 | 36.8 | 45.23 | 7/4/14 |
| 1005 | 28.77 | 31.7 | 26.33 | 7/4/14 |
| 1006 | 34.5 | 28.93 | 29.73 | 7/7/14 |
| 1008 | 34.97 | 31.57 | 30.23 | 7/7/14 |

| | (%5 VINEGAR) | | | |
|---|---|---|---|---|
| SUBJECT | INITIAL MEASUREMENT | AFTER 5 MIN | AFTER 1 HOUR | DATE |
| 1005 | 34.4 | 29 | 35.83 | 7/7/14 |
| 1007 | 52.2 | 50.13 | 56.87 | 7/7/14 |
| 1008 | 38.47 | 30.97 | 41.93 | 7/7/14 |
| 1009 | 15.5 | 15.23 | 18.2 | 7/7/14 |
| 1011 | 51.33 | 48.13 | 52.6 | 7/7/14 |
| 1012 | 16.7 | 15.8 | 18.37 | 7/7/14 |
| 1014 | 22.8 | 21.8 | 23.97 | 7/7/14 |

The inventor has further discovered that this property of vinegar to reduce or eliminate skin drying is enhanced by vinegar infusion of aloe vera, comfrey or mucilage containing plants such as mallow or colt's foot prior to saponification (or alternatively after saponification in some formulations, such as with a neutralized vinegar infusion).

### Effectiveness Against High pH

An over-based soap can have a relatively high pH between about 10.50 - 11.00, which can be a caustic environment for skin. The present invention solves the problem of a high pH environment for skin by lowering the pH of the produced soap base to acceptable levels between about pH 9.0 - pH 10.5 with the addition of natural vinegar to an oil prior to the saponification reaction. Vinegar has natural acidity, characterized mainly by its content of acetic acid, and the addition of the weak acid vinegar effectively reduces the pH level of the produced soap after saponification, thereby providing a milder environment for the skin when using the soap. The skin adapts to reasonable alkalinity much more easily than to a lowered pH environment from a composition of synthetic ingredients, harsh chemicals that do not exist in nature. A vinegar additive or a vinegar infusion of lemon, orange, other citrus fruit(s), or a combination thereof, prior to or after saponification, may be used to further lower the pH of the produced soap base.

### Effectiveness Against Microbial Infection / Allergy

Soaps may have a pH value of above 9.5, and even above 10 for over-based soaps as noted above. At such alkaline levels a bacterial preservative is not needed. However, when the pH is reduced to provide a less harsh environment for skin as described above, the reduced level of pH also increases the risk of bacterial attack. The present invention addition of vinegar to oil(s) prior to saponification provides for the formation of sodium acetate, which is formed when the acetic acid in vinegar reacts with a part of the lye. Advantageously, the sodium acetate functions as an effective natural antimicrobial agent and increases the resistance of the natural soap product to bacterial attack.

Experiments were conducted to determine the effectiveness of the present vinegar additive to soap in preventing microbial infection. Liquid soap samples with and without vinegar were tested for microbial activity employing quantitative analysis methods. 14 ml of undiluted liquid soap samples, both a control set without vinegar and a sample set with 5 wt% vinegar, were incubated at 37±1 degrees Celsius for 4 days. The samples were then examined according to ISO methods EN ISO 21149, EN ISO 22717, EN ISO 22718, EN ISO 18416, EN ISO 21150, and EN ISO 16212. For the control samples with no vinegar at a 9.8 pH value, 10 cfu/ml of gram-negative coccus bacterial growth was observed, but no pathogen microorganisms were observed. In the liquid soap samples whose pH had been lowered to 8.8 by the addition of 5 wt% vinegar, which would be expected to be a more convenient medium of growth for bacteria due to lower pH levels, no bacterial activity was observed.

The inventor has further discovered that this antimicrobial property of vinegar can be enhanced by vinegar infusion of thyme.

The inventor has further discovered that a natural vinegar infusion of stinging nettle, rosewood, and/or chamomile, enhances a function of vinegar to render the soap base non-allergic or less allergic to the skin.

### Advantages

Thus, the soap base of the present invention, formed from a saponification reaction between (1) a first mixture of oil and vinegar and (2) a second mixture of an alkaline solution, provides for many advantages over the prior natural soaps and synthetic soaps produced for consumers.

The soap base of this invention enables petroleum based surface active materials to be effectively replaced by renewable natural sources, as vinegar and all natural components infused into vinegar are renewable natural products. The present invention thus eliminates the need for many petrochemicals that function as antimicrobials, antioxidants and humectants in cosmetic, personal care and household products.

Detergents may accumulate in underground waters and may constitute a threat to the future of mankind. A soap composition could also harm underground waters to some extent if synthetic chemicals of prior consumer synthetic soaps accumulate. By using vinegar alone or as an infusion to make a natural soap that overcomes prior disadvantages, the present invention contributes to maintaining healthy ecosystems as well as to the health of individuals.

The improved natural soap base can be used as a raw material in various industries as a surface-active material with its additional antibacterial and emolient attributes. Advantageously, the infusion technique allows in situ extraction of plant components by vinegar without using chemical solvents. Furthermore, disagreeable odor from the produced soap base composition is removed by adding a vinegar additive to oil prior to the saponification reaction, thereby providing a soap base composition substantially without natural odor from vinegar.

Thus, many of the shortcomings of a natural soap base and products produced therefrom, such as liquid natural soaps, cleaning compositions, personal care products, household products or cosmetic products, have been solved by the addition of a natural component to oil prior to saponification: vinegar or a vinegar infusion of one or more herbs that serve to enhance the function and properties of vinegar. Thus, the soap base of the present invention and products produced therefrom are effective in hard water, effective against producing soap scum, resistant to bacterial infection and oxidative degradation, effective in providing an anti-allergic property and humectant to the skin, effective in providing a milder soap with a lower pH value than ordinary natural soaps, and overall effective in providing an environmentally-friendly "green" soap product which is bioderived, biodegradable, and natural.

The presence of a vinegar component in a soap composition, such as for soap bases, soaps, and other products produced from such a soap composition, have the above-explained advantages. However, the use of vinegar may cause an odor disliked by users of the soap composition product.

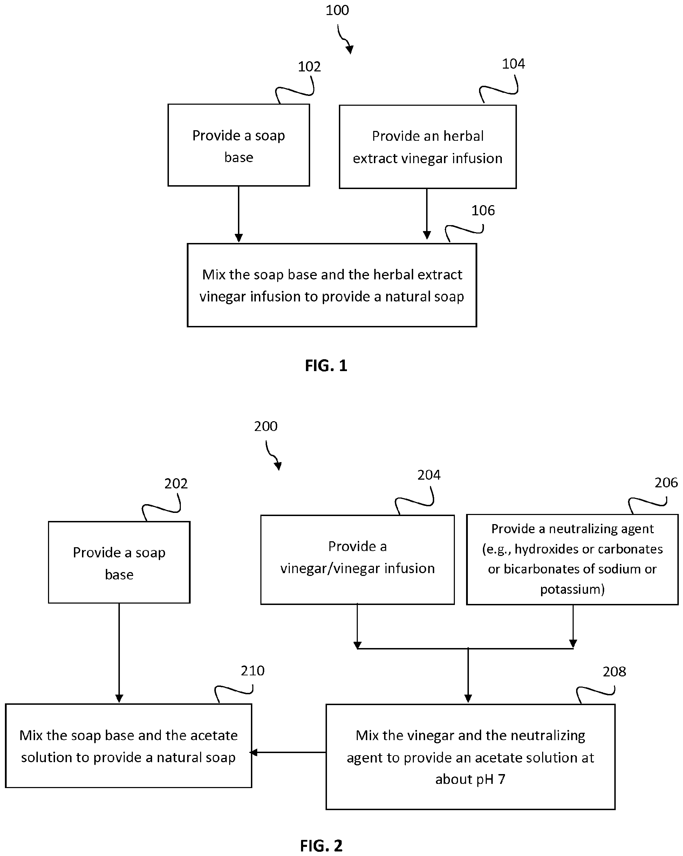

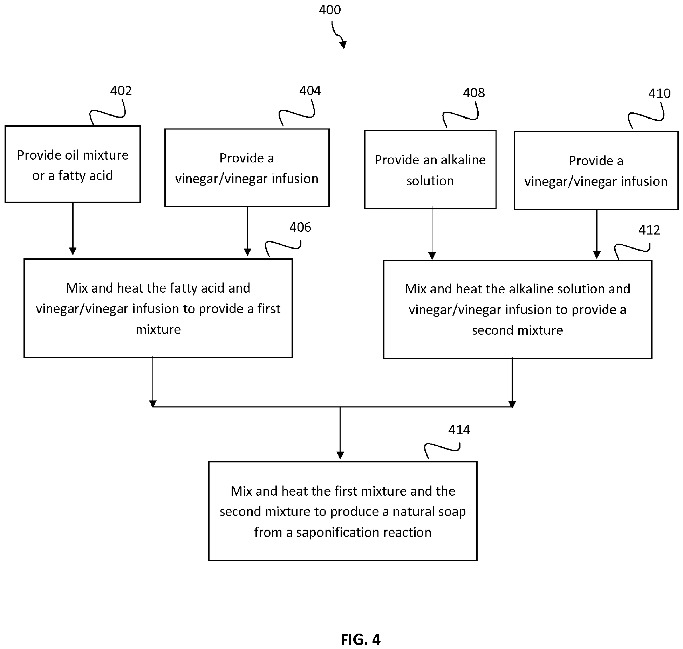

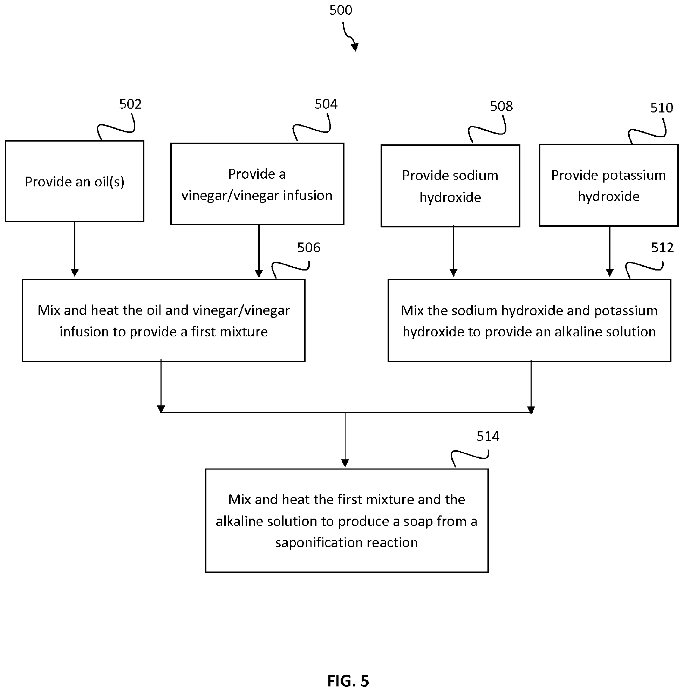

Referring now FIGS. 1 to 5, three methods are described herein to overcome the odor issue and other disadvantages of traditional natural soaps, in accordance with embodiments of the present invention.

FIG. 1 illustrates a method 100 in which adding an infusion or a decoction of a plant in vinegar to water with which a soap base is diluted into a liquid soap, or other product, substantially "hides" vinegar odor. In other words, an herbal extract vinegar infusion including an extract of a plant additive, such as calendula, that masks or hides the vinegar odor, may be used to overcome the vinegar odor issue and the disadvantages of traditional natural soap. Furthermore, an herbal extract vinegar infusion enables the incorporation of desired ingredients of plants into the product without the need for an external solvent and subsequent solvent removal.

Method 100 includes providing a soap base at step 102, providing an herbal extract vinegar infusion at step 104, and then mixing the soap base and the herbal extract vinegar infusion to provide a natural soap at step 106, in accordance with an embodiment of the present invention.

FIG. 2 illustrates a method 200 in which adding "neutralized" vinegar can be used to overcome vinegar odor and the disadvantages of traditional natural soap. Vinegar is neutralized by the addition of hydroxides, carbonates, or bicarbonates of sodium or potassium to convert the acetic acid of vinegar to an acetate. The neutralized vinegar may then be added to deionized water with which a soap base is diluted into a liquid soap, or other product.

Method 200 includes providing a soap base at step 202, providing a vinegar or vinegar infusion at step 204, and providing a neutralizing agent at step 206 (e.g., hydroxides or carbonates or bicarbonates of sodium or potassium). The vinegar or vinegar infusion is mixed with the neutralizing agent at step 208 to provide an acetate solution, for example at about pH 7. Then, at step 210, the soap base and the acetate solution are mixed to provide a natural soap in accordance with an embodiment of the present invention.

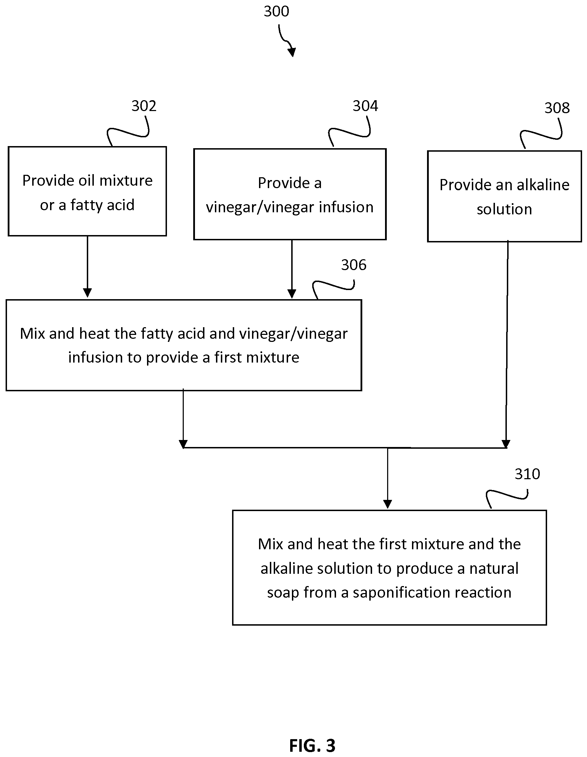

FIGS. 3 to 5 illustrate methods in which adding vinegar prior to a saponification reaction, and not to the soap after the saponification process, can be used to overcome vinegar odor and the disadvantages of traditional natural soap.

A method 300 includes providing an oil mixture or a fatty acid mixture (e.g., from oil or other fatty acid source) at step 302, providing a vinegar or vinegar infusion at step 304, and mixing and heating the oil or fatty acid and the vinegar or vinegar infusion to provide a first mixture at step 306. An alkaline solution is provided at step 308, and then the first mixture and the alkaline solution are mixed to produce a natural soap from a saponification reaction at step 310, in accordance with an embodiment of the present invention.

A method 400 includes providing an oil or a fatty acid (e.g., from oil or other fatty acid source) at step 402, providing a vinegar or vinegar infusion at step 404, and mixing and heating the oil or fatty acid and the vinegar or vinegar infusion to provide a first mixture at step 406. Method 400 further includes providing an alkaline solution at step 408, providing another vinegar or vinegar infusion (can be the same or a different vinegar component as in step 404) at step 410, and mixing and heating the alkaline solution and the vinegar or vinegar infusion to provide a second mixture at step 412. Then the first mixture and the second mixture are mixed to produce a natural soap from a saponification reaction at step 414, in accordance with an embodiment of the present invention. In this method, a vinegar component is shown to be added to both a fatty acid reactant and an alkaline solution reactant prior to saponification.

A method 500 includes providing an oil at step 502, providing a vinegar or vinegar infusion at step 504, and mixing and heating the oil and the vinegar or vinegar infusion to provide a first mixture at step 506. Method 500 further includes providing sodium hydroxide at step 508, providing potassium hydroxide at step 510, and mixing the sodium hydroxide and the potassium hydroxide to provide an alkaline solution at step 512. Then the first mixture and the alkaline solution are mixed to produce a natural soap from a saponification reaction at step 514, in accordance with an embodiment of the present invention. In this method, the alkaline solution can be a mixture of sodium hydroxide and potassium hydroxide at various ratios. In one example, the ratio is 1 mol sodium hydroxide to 2 mol potassium hydroxide. In another example, the mole ratio of sodium to potassium as a fatty acid counter ion is in a range of 1:1 to 1:2.

### Vinegar Soap Composition Examples

A further understanding of embodiments of the present invention can be obtained by reference to certain specific examples for preparation of soap bases in accordance with the present invention, which are provided herein for purposes of illustration only and are not intended to be limiting. Vinegar (or a vinegar infusion of herbal extract) is added to an oil component before saponification. In other embodiments, vinegar or a vinegar infusion can also be added to water which dilutes the soap base to produce a final product.

### Example 1

An example soap base was produced as follows:
85 grams of olive oil, 10 grams of coconut oil, and 5 grams of castor oil were mixed and heated to about 80 degrees Celsius to form an oil mixture. 4.46 grams of sodium hydroxide and 12.49 grams of potassium hydroxide were dissolved in 39.6 grams of deionized water to form an alkaline solution. The alkaline solution was added to the oil mixture. By continuous mixing and moderate heating for about 4 hours, a soap gel was formed. This soap base was made to rest over-night.

### Example 2

A liquid hand soap in accordance with an embodiment of the present invention was produced as follows:
10 grams of natural apple cider vinegar was dissolved in 150 grams of deionized water and neutralized to pH 7 by the addition of hydroxides, carbonates, or bicarbonates of sodium or potassium to form an acetate solution. Aromatic or functional essential oils were added to this acetate solution. The soap base in example 1 was diluted with this acetate solution to form a natural soap composition in accordance with an embodiment of the present invention.

### Example 3

An example vinegar infusion in accordance with an embodiment of the present invention was prepared as follows:
2 grams of dry Calendula Officinalis was placed into 10 grams of apple cider vinegar and boiled for 5 minutes. This enabled the extraction of active materials from the calendula, such as luteolin, kaempferol, and apigenin, into the vinegar. This vinegar infusion of calendula was filtered to remove calendula residue and the vinegar infusion was then diluted with 150 grams of deionized water.

Aromatic or functional essential oils were dissolved, if desired by mechanical homogenization or sonication, in glycerol and added to the solution of vinegar infusion and water. The soap base in example 1 was diluted with this solution of vinegar infusion and water to form a natural soap composition in accordance with an embodiment of the present invention.

It is noted that dissolving essential oils in glycerol prior to adding to the solution of vinegar infusion and water enables better dispersion of the oils, thereby preventing the oils floating on the liquid soap surface and better phase stability.

### Example 4

An example soap base in accordance with an embodiment of the present invention was produced as follows:
85 grams of olive oil, 10 grams of coconut oil, 5 grams castor oil, and 10 grams of apple cider vinegar were mixed and heated to about 80 degrees Celsius to form a first mixture. 4.6 grams of sodium hydroxide and 12.77 grams of potassium hydroxide were dissolved in 40.4 grams of deionized water to form an alkaline solution. The alkaline solution was added to the first mixture. By continuous mixing and moderate heating for about 4 hours, a natural soap gel was formed in accordance with an embodiment of the present invention. This soap base was made to rest overnight.

### Example 5

An example soap base was produced as follows:
85 grams of olive oil, 10 grams of coconut oil, and 5 grams of castor oil were mixed and heated to about 80 degrees Celsius to form a first mixture. 5.67 grams of sodium hydroxide and 13.36 grams of potassium hydroxide were dissolved in 44.4 grams of deionized water to form a second mixture. The second mixture (alkaline solution) was added to the first mixture (the oil mixture). By continuous mixing and moderate heating for about 4 hours, a soap gel was formed. This soap gel was made to rest over-night.

### Example 6

An example liquid hand soap in accordance with an embodiment of the present invention was produced as follows:
24.7 grams of natural apple vinegar infusion of comfrey (a mucilage containing plant) was dissolved in 305 grams of deionized water and aromatic or functional essential oils were added to the vinegar solution. The soap base of Example 5 was then diluted with this solution of vinegar, oils, and water to form a natural soap composition in accordance with an embodiment of the present invention.

### Example 7

An example natural shampoo in accordance with an embodiment of the present invention was produced as follows:
5 grams of laurel leaf essential oil and 10 grams of apple vinegar infusion of stinging nettle were added to 318 grams of deionized water. The soap base in Example 5 was diluted with this solution of laurel leaf essential oil, apple vinegar infusion of stinging nettle, and water, to produce a shampoo in accordance with an embodiment of the present invention. This shampoo carried the known positive effects of stinging nettle and laurel leaves to the hair. The vinegar made the hair shiny and the shampoo was more easily rinsed from the hair.

### Herbal Extraction Methods

Methods of herbal extraction in accordance with the present invention, production process steps, and products formed using such methods, substantially eliminate or reduce the disadvantages of the prior traditional herbal extraction methods described above. In one embodiment, the present invention describes the use of one or more raw materials of a soap composition product, separately or in combination, as extraction media for herbs of interest for eliminating or substantially reducing the above-noted problems of herbal extraction in industry. In other words, herbal components of interest are incorporated into end products by means of using only their raw material(s) as a solvent(s), thereby eliminating the need to separate solutes from solvents.

In one embodiment, a soap base composition is produced from a saponification reaction between: (a) a first mixture including a first herbal extract infusion having soluble components of an herb solvated in a first extraction medium, wherein the first extraction medium is a first saponification reactant for producing a soap base composition; and (b) a second mixture including a second saponification reactant for producing the soap base composition.

In another embodiment, a soap base composition is produced from a saponification reaction between: (a) a first mixture including a first herbal extract infusion having soluble components of a first herb solvated in a first extraction medium, wherein the first extraction medium is a first saponification reactant for producing the soap base composition, the first extraction medium selected from the group consisting of vinegar, turpentine, oil, fat, milk, glycerin, water, and any combination thereof; and (b) a second mixture including a second herbal extract infusion having soluble components of a second herb solvated in a second extraction medium, wherein the second extraction medium is a second saponification reactant for producing the soap base composition, the second extraction medium selected from the group consisting of sodium hydroxide, potassium hydroxide, and any combination thereof.

In the above embodiments, the following alternatives may be combined in various applicable and functioning combinations within the scope of the present invention. In one alternative, the first herbal extract infusion includes a first extraction medium selected from the group consisting of vinegar, turpentine, oil, fat, milk, sodium hydroxide, potassium hydroxide, glycerin, water, and any combination thereof. In another alternative, the second mixture includes a second herbal extract infusion having soluble components of an herb solvated in a second extraction medium, wherein the second extraction medium has a same composition as the second saponification reactant.

In one alternative, the herb solvated in the second extraction medium may be the same herb solvated in the first extraction medium, the herb solvated in the first extraction medium undergoing multiple extractions in different extraction media. In another alternative, the herb solvated in the first extraction medium undergoing multiple extractions in different extraction media is selected from the group consisting of sunflower seeds, almonds, hazelnuts, corn, olive and olive kernels, and palm and palm kernels.

In another alternative, the second saponification reactant may be an alkaline hydroxide including one of a sodium hydroxide, a potassium hydroxide, and any combination thereof.

In another alternative, the soap base composition may have a pH value between about 9 and about 10.5.

In another alternative, a saponification reaction may take place at a temperature between 70 degrees Celsius and 99 degrees Celsius.

In another alternative, the first extraction medium is vinegar and the herb solvated in vinegar is selected from the group consisting of:
Calendula (Calendula officinalis),
Nettle (Urtica dioica),
Green tea (Camellia sinensis),
Horsetail (Equisetum arvense),
Lavender (lavendula officinalis),
Rosemary (Rosmarinus officinalis),
Sage (Salvia officinalis),
Melissa (Melissa officinalis),
Olive leaves (Olea europaea),
Thyme (Thymus vulgaris),
Carob (Ceratonia siliqua),
Dandelion (Taraxacum officinale),
Cinnamon (cinnamomum zeylanicum),
Cloves (sysygium aromaticum),
Redclover (Trifolium pratense),
Motherwort (Leonurus cardiaca),
Burdock (Arctium lappa),
Tarragon(Artemisia dracunculus),
Basil (Ocimum basilicum),
Mint (Mentha piperita),
Dill (Anethum graveolens),
Chamomile flowers (Matricaria chamomilla),
Ginseng (Panax ginseng),
Alfalfa (Medicago sativa),
Citrus peels (citrus),
Yarrow (Achillea millefolium),
Licorice (Glycyrrhiza glabra),
Digitalis (Digitalis purpurea),
Gentian root (Gentiana Lutea),
Yellow dock (Rumex crispus),
Huang Qi (Astragalus membranaceus),
Blue cocosh (Caulophyllum thalictroides),
Wild yam (Dioscorea villosa),
Pokeroot(Phytolacca americana),
Senega root (Polygala senega),
Comfrey (Symphytum officinale),
Mullein (Verbascum Thapsus),
Cayenne (Capsicum),
Artichoke (Cynara scolymus),
Male fern (Dryopteris filix-mas),
Meadowsweet (Filipendula ulmaria),
Wintergreen (Gaultheria Procumbens),
Willow bark (Salix),
Goldenrod (Solidago virgaurea),
Skunk cabbage (symplocarpus Foetidus),
Feverfew (Tanacetum parthenium),
Thuja (Thuja occidentalis),
Linden (Tilia),
Coltsfoot (Tussilado farfara),
Valerian (Valeriana officinalis),
Vervain (Verbena officinalis),
Periwinkle (Vinca major),
Violet (Viola Odorata),
Pansy (Viola tricolor),
Corn (Zea mays L.),
Ginger (Zingiber officinale), and
any combination thereof.

In another alternative, the first extraction medium is turpentine and the herb solvated in turpentine is selected from the group consisting of:
Rosemary (Rosmarinus officinalis),
Black cumin (Nigella sativa),
Citrus peel (citrus),
Sunflower (Helianthus annuus),
Bayberry (Myrica Cerifera),
Cloves (sysygium aromaticum),
Tarragon (artemisia dracunculus),
Vanilla (Vanilla planifolia),
Cinnamon (cinnamomum zeylanicum),
Borage (Borago officinalis),
Burdock (Arctium lappa),
Cedar (Cedrus atlantica),
Mint (Mentha piperita),
Comfrey (Symphytum officinale),
Echinacea (Echinacea angustifolia),
Elderberry (Sambucus nigra),
Nettle (Urtica dioica),
Eucalyptus (Eucalyptus globulus),
Ginkgo (Ginkgo biloba),
Horehound(Marrubium vulgare),
Sage (Salvia officinalis),
Marshmallow (althaea officinalis),
Rhubarb (Rheum officinale),
St John's wort (Hypericum perforatum),
Thyme (Thymus vulgaris),
Witch hazel (Hamamelis virginiana),
Yarrow (Achillea millefolium),
Acacia (Acacia Senegal),
Tragacanth (Astragalus gummifer),
Irish moss (Chondrus crispus),
Cluster beans (Cyamopsis tetragonoloba),
Quince leaves(Cydonia Oblonga),
Psyllium (plantago ovata),
Fenugreek (Trigonella Foenum-Graecum),
Coltsfoot (Tussilado farfara),
Slippery elm (ulmus rubra),
Shallaki (Boswellia serrata),
Camphor laurel (Cinnamomum Camphora),
Myrrh (Commiphora molmol),
Dragon's blood (Daemonorops draco),
Grindelia (Grindelia),
Lignum vitae (Guaiacum officinale),
Dandelion (Taraxacum officinale),
Tansy (Tanacetum vulgare),
Rue (Ruta graveolens),
Bogbean (Menyanthes Trifoliata),
Goldenseal (Hydrastis Canadensis),
Gentian root (Gentiana Lutea),
Boneset (Eupatorium perfoliatum),
Centaury (Erythraea centaurium),
Barberry (Berberis vulgaris), and
any combination thereof.

In another alternative, the first extraction medium is plant oil and the herb solvated in plant oil is selected from the group consisting of:
Calendula (Calendula officinalis),
Lavender (Lavendula officinalis),
Sage (Rosmarinus officinalis),
Yarrow (Achillea millefolium),
Burdock (Arctium lappa),
Licorice (Glycyrrhiza glabra),
Ginseng (Panax ginseng),
Dandelion (Taraxacum officinalis),
Digitalis (Digitalis purpurea),
Gentian root (Gentiana Lutea),
Yellow dock (Rumex crispus),
Huang Qi (Astragalus membranaceus),
Blue cocosh (Caulophyllum thalictroides),
Wild yam (Dioscorea villosa),
Pokeroot (phytolacca americana),
Senega root (Polygala senega),
Comfrey (Symphytum officinale),
Mullein (Verbascum Thapsus),
Marshmallow (althaea officinalis),
Arnica (Arnica Montana),
Eucalyptus (Eucalyptus globulus),
Goldenseal (Hydrastis Canadensis),
Elderberry (Sambucus nigra),
Chickweed (Stellaria media),
Thyme (Thymus vulgaris),
Slippery elm (Ulmus rubra),
Malva (Malva sylvestris),
Cranesbill (Geranium maculatum),
Tea tree(Melaleuca), and
any combination thereof.

In another alternative, the first extraction medium is milk and the herb solvated in milk is selected from the group consisting of:
Tamarind (Tamarindus indica),
Olive leaves (Olea europaea),
Cloves (sysygium aromaticum),
Cinnamon (cinnamomum zeylanicum),
Thyme (Thymus vulgaris),
Quince leaves (Cydonia Oblonga),
Yarrow (Achillea millefolium),
Horse chestnut (Aesculus hippocastanum),
Buchu (Agathosma betulina),
Agrimony (Agrimonia eupatoria),
Parsley piert (Alchemilla arvensis),
Marshmallow (Althaea officinalis),
Dill (Anethum graveolens),
Angelica (Angelica archangelica),
Celery (apium graveolens),
Wormwood(Artemisia absinthium),
Oat (Avena Sativa),
Black horehound (Ballota nigra),
Calendula (Calendula officinalis),
Shepherd's-purse (Capsella bursa-pastoris),
Cayenne pepper (Capsicum annuum),
Cummin (Carum Carvi),
Iceland moss (Cetraria islandica),
Irish moss (Chondrus crispus),
Hawthorn (Crataegus laevigata),
Broom (Cytisus scoparius),
Carrot (Daucus carota),
Sundew (Drosera rotundifolia),
Couch grass (Elymus repens),
Ma huang (Ephedra sinica),
California poppy (Eschscholtzia Californica),
Eucalyptus (Eucalyptus globulus),
Boneset (Eupatorium perfoliatum),
Eyebright (Euphrasia),
Meadowsweet (Filipendula ulmaria),
Fennel (Foeniculum vulgare),
Fumitory (Fumaria officinalis),
Goat's rue (Galega officinalis),
Cleavers (Galium aparine),
Cranesbill (Geranium maculatum),
Ginkgo (Ginkgo biloba),
Grindelia (Grindelia),
Mouse-ear hawkweed (Hieracium pilosella),
Hop (Humulus lupulus),
St John's wort (Hypericum perforatum),
Hyssop (Hyssopus Officinalis),
Juniper (Juniperus Communis),
Wild lettuce (Lactuca Virosa),
Lavender (Lavendula officinalis),
Motherwort (Leonurus cardiaca),
Flax (Linum usitatissimum),
Bugleweed (Lycopus europaeus),
Malva (Malva sylvestris),
Horehound (Marrubium vulgare),
Chamomile flowers (Matricaria chamomilla),
Melissa (Melissa officinalis),
Mint (Mentha piperita),
Pennyroyal (Mentha pulegium),
Wintergreen (Mitchella repens),
Catnip (Nepeta cataria),
Passion flower (Passiflora incarnata),
Parsley (Petroselinum Crispum),
Boldo (Peumus boldus),
Pokeroot (Phytolacca americana),
Anise (Pimpinella anisum),
Plantain (Plantago major),
Senega root (Polygala senega),
Pulsatilla (Pulsatilla vulgaris),
Rosemary (Rosmarinus officinalis),
Raspberry (Rubus idaeus),
Rue (Ruta graveolens),
Sage (Salvia Officinalis),
Elderberry (Sambucus nigra),
Figwort (Scrophularia nodosa),
Skullcap (Scutellaria Lateriflora),
Senna (Senna),
Saw palmetto (Serenoa repens),
Goldenrod (Solidago virgaurea),
Chickweed (Stellaria media), and
any combination thereof.

In another alternative, the first extraction medium is glycerol and an herb solvated in glycerol is selected from the group consisting of the following herbs:
Rosmarinic acid containing plants:
Agastache rugosa Korean mint
Anethum graveolens dill
Artemisia dracunculus Tarragon
Borago officinalis Borage
Collinsonia canadensis Canada Horsebalm
Glechoma hederacea ground-ivy
Hyssopus officinalis hyssop
Lavandula angustifolia lavander
Lavandula x intermedia lavandin
Lavandula latifolia Portuguese lavender
Lycopus europaeus gypsywort
Lycopus virginicus
Melissa officinalis lemon balm
Mentha pulegium pennyroyal
Mentha spicata Spearmint
Mentha longifolia Horse Mint
Mentha arvensis var. piperascens corn mint
Mentha aquatica water mint
Mentha x piperita Peppermint
Mentha x rotundifolia
Momordica charantia bitter melon
Monarda fistulosa Wild bergamot
Monarda didyma
Nepeta catariacatnip
Ocimum tenuiflorum holy basil
Ocimum basilicum basil
Origanum vulgare Oregano
Origanum majorana Marjoram
Perilla frutescens
Petroselinum crispum
Prunella vulgaris common self-heal
Rosmarinus officinalis rosemary
Salvia officinalis Sage tea
Salvia sclarea clary sage
Satureja hortensis Summer savory
Satureja montana Winter savory
Scrophularia scorodonia figworts
Stachys officinalis hedgenettle
Symphytum officinale common comfrey
Teucrium scorodonia wood sage
Thymus vulgaris German thyme
Thymus serpyllum wild thyme
Vaccinium corymbosum high blueberry
Carnosic acid containing plants:
Hyptis dilatata
Lepechinia meyenii
Lepechinia hastata
Ocimum tenuiflorum holy basil
Pulicaria salviaefolia
Rosmarinus officinalis rosemary
Salvia munzii Munz's sage
Salvia canariensis Canary Island sage
Salvia apiana white sage
Salvia mellifera black sage
Salvia willeanaTroodos Sage
Salvia officinalis Sage tea
Salvia tomentosa
Salvia columbariae chia sage
GENISTEIN containing plants:
Adenocarpus foliolosus
Adenocarpus decorticans
Albizia procera
Amphicarpaea edgworthii
Amphicarpaea bracteata hog peanut
Apios americana
Argyrocytisus battandieri
Baptisia sp
Baptisia tinctoria Wild Indigo
Bowdichia nitida
Cajanus cajan Pigeonpea
Cajanus scarabaeoides
Calicotome villosa
Calicotome spinosa
Calopogonium caeruleum
Camptosema sp
Canavalia eurycarpa
Canavalia rosea
Canavalia ensiformis Jack Bean
Canavalia virosa
Canavalia gladiata Kachang polong; Carabanz; Kachang parang (Chopper bean)
Canavalia galeata
Canavalia sericea
Centrosema schiedianum
Centrosema pascuorum
Centrosema sagittatum
Centrosema plumieri
Chamaecytisus supinus
Chamaecytisus hirsutus
Chamaecytisus smyrnaeus
Chamaecytisus eriocarpus
Chamaecytisus ratisbonensis
Chamaecytisus albus
Chamaespartium tridentatum
Chamaespartium sagittale
Chronanthus biflorus
Cicer arietinum Chickpea; Garbanzo
Clitoria ternateaKembang teling; Mazeryon Hindi; Menteling; Cwetabhanda
Clitoria falcata
Cologana broussonetii
Crotalaria juncea Sunhemp
Cytisus multiflorus
Cytisus tribracteolatus
Cytisus ingramii
Cytisus scoparius Scotch Broom
Cytisus striatus
Cytisus ardoini
Desmodium gangeticum Serengan; Vidarigandha; Lepantan; Akar katuh; Kemani bali;
Meringan
Dioclea glycinoides
Dipogon lignosus
Dolichos biflorus Kulattha
Dunbaria villosa Wild Yam
Echinospartium horridum
Erinacea anthyllis
Eriosema nutans
Eriosema glomeratum Mampong
Eriosema psoraleoides
Erythrina crista-galli Cockspur Coral Tree
Erythrina sp
Flemingia strobilifera
Galactia jussiaeana
Genista patula
Genista germanica
Genista sp genet
Genista ovata
Genista clavata
Genista tinctoria
Genista rumelica
Genista lydia
Glycine max soybean
Glycine wightii
Glycyrrhiza glabra licorize
Hardenbergia violacea
Kennedia nigricans (Black Kennedia
Kennedia rubicunda dusky coral pea
Kennedia coccinea (coral vine
Kennedia procurrens
Laburnum alpinum Scotch laburnum
Laburnum anagyroides golden rain
Lembotropis emerifolia
Lens culinaris lentil
Lespedeza cyrtobotrya bush clovers
Lupinus albus white lupin
Lupinus luteus European yellow lupine
Lupinus perennis wild perennial lupine,
Lygos raetum
Lygos monosperma
Maackia amurensis
Macroptylium bracteatum
Macroptylium martii
Macroptylium atropurpureum
Macroptylium lathyroides
Macrotyloma axillare
Macrotyloma uniflora
Medicago sativa
Moghania macrophylla
Mucuna deeringiana
Mucuna pruriens
Neonotonia wightii
Ononis spinosa
Pericopsis laxiflora
Phaseolus lunatus
Phaseolus vulgaris
Phaseolus spp
Phaseolus lunatus
Phaseolus coccineus
Piptanthus nepalensis
Piptanthus nanus
Pisum sativum
Podocarpus spicatus
Prunus nipponica
Prunus avium
Prunus aequinoctialis
Prunus verecunda
Prunus maximowiczii
Prunus cerasus
Prunus spp
Prunus mahaleb
Pseudeminia comosa
Pseudoeriosema borianii
Pseudovigna argentea
Psoralea corylifolia
Pterocarpus angolensis
Pueraria pseudohirsuta
Pueraria phaseoloides
Pueraria montana
Rhynchosia densiflora
Rhynchosia pyramidalis
Rhynchosia cabibaea
Rhynchosia phaseoloides
Rhynchosia minima
Rhynchosia acuminatifolia
Rhynchosia volubilis
Rhynchosia hirsuta
Sophora japonica
Sophora subprostrata
Spartium junceum
Stauracanthus genistoides
Stauracanthus boivinii
Stellaria media
Stizolobium deeringianum
Strongylodon macrobotrys
Teline sp
Teyleria koordersii
Thermopsis sp
Thermopsis fabacea
Trifolium subterraneum
Trifolium hybridum
Trifolium pratense
Trifolium sp
Trifolium brachycalycinum
Trifolium repens
Trifolium alexandrinum
Trigonella foenum-graecum
Ulex sp
Ulex micranthus
Vandasina retusa
Vicia faba
Vigna angularis
Vigna unguiculata
Vigna subterranea
Vigna radiata
GENISTIN containing plants:
Genista tinctoria dyer's broom
Glycine max soybean
Prunus cerasus sour cherry
Pueraria montana kudzu
Trifolium pratense red clover
Ulex europaeus common gorse
DAIDZEIN containing plants:
Cicer arietinum Chickpea
Genista tinctoria Dyer's Broom
Glycine max Soybean
Lens culinaris Lentil
Medicago sativa alfalfa
Phaseolus lunatus butter bean
Phaseolus vulgaris black bean
Phaseolus coccineus scarlet runner bean
Pisum sativum pea
Psoralea corylifolia babchi
Pueraria pseudohirsuta chinese kudzu
Pueraria montana kudzu
Sophora subprostrata Shan Dou Gen
Trifolium pratense Peavine Clover
Vicia faba Faba Bean
Vigna radiata Mungbean
Vigna angularis Adzuki Bean
Vigna unguiculata Asparagus Bean
DAIDZIN containing plants:
Glycine max Soybean
Pueraria montana kudzu
Pueraria pseudohirsuta chinese kudzu
Trifolium pratense Peavine Clover
and any combination thereof.

In another alternative, the first herbal extract infusion may include a soluble extract of an herb selected from the group consisting of the herbs listed above or a combination thereof. In another alternative, the second herbal extract infusion may include a soluble extract of an herb selected from the group consisting of the herbs listed above or a combination thereof.

It is noted that the herbal extract infusion may be used without separation from herbal residue or the herbal residue after physical filtering may be used without separation from the extraction medium.

It is further noted that the raw materials or ingredients for a product described above may also be used in combination or consecutively as extraction media.

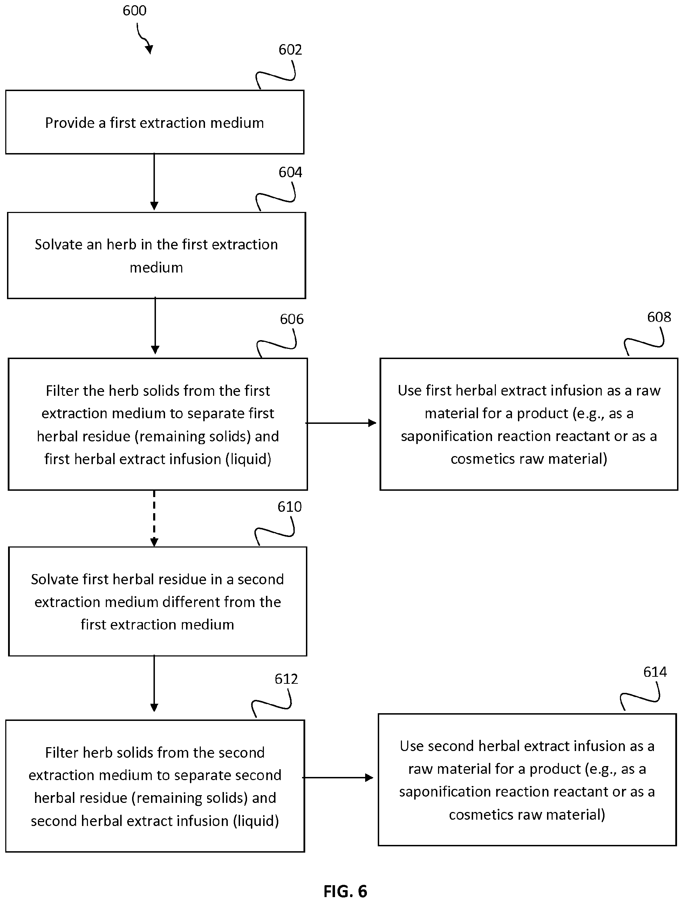

Referring now to FIG. 6, a flowchart is shown of a method 600 for herbal extraction in accordance with an embodiment of the present invention. Method 600 includes providing a first extraction medium at step 602, solvating an herb in the first extraction medium for a time period at step 604, and filtering the herb solids from the first extraction medium to separate a first herbal residue (remaining solids) from a first herbal extract infusion (liquid). At step 608, this first herbal extract infusion is used as a raw material for a product, such as for a saponification reaction reactant for producing a soap composition product or as a raw material for a cream or other cosmetic product.

Optionally, as shown by the dashed arrow to step 610, the first herbal residue from step 606 is solvated in a second extraction medium that is different from the first extraction medium. The herb solids are filtered from the second extraction medium to separate second herbal residue (remaining solids) from the second herbal extract infusion (liquid) at step 612. At step 614, the second herbal extract infusion is used as a raw material for a product. Accordingly, it is evident that further extractions consecutively using herbal residue is within the scope of the present invention. Advantageously, the raw materials and herbs are used in combination and/or consecutively as extraction media in order to fully utilize the raw materials and to fully extract active ingredients from the plant additive/herb.

### Advantages

The present invention eliminates the extra step needed to separate an extract from a solvent used in the extraction process, as the solvent itself is a raw material of the end product that is produced. This also eliminates the risk of degradation of the active ingredients in the extracts since high temperatures for solvent separation are avoided. Extracts that are produced in the production area in necessary amounts when needed are more fresh and potent than those produced by other methods or requiring lengthy storage times.

Thus, the present invention provides for production in simpler extraction facilities with lower costs of investment, stock and operations when utilizing herbal extracts in the production of soap, household, cosmetics, and like products. For example, herbal extraction as described above may take place in the production area.

Herbal remnants of extraction in natural products can also be used safely as animal food in the animal feedstock sector.

### Herbal Extraction Examples

Having generally described the present invention, a further understanding can be obtained by reference to certain specific examples for using a raw material herbal extraction medium in accordance with the present invention, which are provided herein for purposes of illustration only and are not intended to be limiting.

### Example 1

Vinegar, a raw material for a soap base composition or a cosmetic product, was used as an herbal extraction medium as follows:
One unit of dried horsetail (Equisetum arvense) was soaked in four units of apple cider vinegar for 2-6 weeks. After filtering solids from the horsetail and vinegar mixture, the vinegar infusion of horsetail was used to produce a soap base. The soap base was used to produce a shampoo (liquid soap). Active ingredients found in the horsetail, such as cysteine, selenium, and zinc were carried to the shampoo by the vinegar infusion

One unit of dried and granulated soy bean (Glycine max) was soaked in four units of apple cider vinegar for 15 minutes. After filtering solids from the soy bean and vinegar mixture, the vinegar infusion of soy bean was used to produce a soap base. The soap base was used to produce a shampoo (liquid soap).

Active ingredients found in the soy bean, such as daidzin and genistin, were carried to the shampoo by the vinegar infusion, to produce a shampoo that helps thicken hair strands, prevent hair loss, regain hair color, and prevent dandruff.

Vinegar functioned better as an extraction medium for daidzin and genistin from soy bean than solvents such as hexane or ethyl alcohol. Table 5 below illustrates some comparative results of extraction analyzed by liquid chromatography (LC).

**Table 5. Soy Bean Extraction**

| **No** | **Extracts** | **Daidzin(mg/L)** | **Genistin (mg/L)** |
|---|---|---|---|
| **1** | Soya-Ethyl Alcohol | 16.51 ± 0.14 | 15.04 ± 0.13 |
| **2** | Soya-Hexane | 00.00 ± 0.00 | 00.00 ± 0.00 |
| **3** | Soya-Vinegar | 42.51 ± 0.14 | 34.78 ± 0.29 |

### Example 2

Turpentine, a raw material for shampoo, was used as an herbal extraction medium as follows:
Rosemary (Rosmarinus officinalis) was added to turpentine in a ratio of 1:4, and the mixture was heated in a hot water bath for 4 hours. After filtering solids from the rosemary and turpentine mixture, the turpentine infusion of rosemary was used as a raw material to produce a shampoo.

Active ingredients of rosemary, such as alpha-pinene and carnosol, were carried to the shampoo by the turpentine infusion, to produce a shampoo that helps prevent dandruff and supports the formation of healthy hair follicles. Turpentine helps to open pores and heal skin on the scalp.

Turpentine functioned better as an extraction medium for carnosol from rosemary than solvents such as water, ethyl alcohol, and hexane. Table 6 below illustrates some comparative results of extraction analyzed by high performance liquid chromatography (HPLC).

**Table 6. Rosemary Extraction**

| **No** | **Extracts** | **Carnosol(mg/L)** |
|---|---|---|
| **1** | Rosemary-Water | 0.79 ± 0.05 |
| **2** | Rosemary-Ethyl Alcohol | 18.95 ± 0.05 |
| 3 | Rosemary-Hexane | 4.87 ± 0.02 |
| **4** | Rosemary-Turpentine | 29.02 ± 0.02 |

### Example 3

Oil, a raw material for a soap base or skin care product, was used as an herbal extraction medium as follows:
Calendula (Calendula officinalis) was soaked in sunflower oil for 1 month. After filtering solids from the calendula and oil mixture, the oil infusion of calendula was used as a raw material in the production of a soap or skin care product. Active ingredients of calendula, such as fat soluble vitamins, antioxidants, resins, and saponins, were carried into the soap or skin care product by the oil infusion, to produce a soap or skin care product that helps to moisturize and heal skin, treat sun spots, reduce wrinkles, and act as an astringent.

Calendula was also mixed in olive oil for 15 minutes to test extraction of active substances, such as luteolin, apigenen, and kaempferol. Olive oil functioned better as an extraction medium for kaempferol from calendula than solvents such as ethyl alcohol and hexane. Table 7 below illustrates some comparative results of extraction analyzed by HPLC.

**Table 7. Calendula Extraction**

| **No** | **Extracts** | **Kaempferol(mg/L)** |
|---|---|---|
| **1** | Calendula-Ethyl Alcohol | 2.10 ± 0.00 |
| **2** | Calendula-Hexane | 2.20 ± 0.00 |
| **3** | Calendula-Olive Oil | 7.10 ± 0.00 |

### Example 4

Glycerin and water, a raw material for a skin care product, was used as an herbal extraction medium as follows:
Witch hazel (Hamamelis virginiana) was soaked in a 75:25 mixture of vegetable glycerin and water at a 1:4 ratio, and heated in a hot water bath for 4 hours. After filtering solids from the witch hazel, glycerin, and water mixture, the glycerin and water infusion of witch hazel was used as a raw material in a skin care product. Active ingredients of witch hazel, such as gallotannins, were carried into the skin care product by the glycerine and water infusion, to produce a skin care product that acts as a sclerotherapeutic and astringent, thus healing spider veins, varicose veins, and eczema.

Soya bean was also soaked in a 2:1 mixture of glycerine and water at a 1:4 ratio by weight, and heated in a hot water bath for 4 hours. After filtering solids from the soya bean, glycerin, and water mixture, the glycerin and water infusion of soya bean were used in a cream manufacture.

The glycerin-water mixture functioned better as an extraction medium for daidzein and genistein from soy bean than solvents such as ethyl alcohol and hexane. Table 8 below illustrates some comparative results of extraction analyzed by liquid chromatography-mass spectroscopy (LC-MS).

**Table 8. Soy Bean Extraction**

| **No** | **Extracts** | **Daidzein(mg/L)** | **Genistein (mg/L)** |
|---|---|---|---|
| **1** | Soya Bean - Ethyl Alcohol | 4.45 ± 0.04 | 3.38 ± 0.03 |
| **2** | Soya Bean -Hexane | 00.00 ± 0.00 | 00.00 ± 0.00 |
| **3** | Soya Bean-Vinegar | 3.72 ± 0.03 | 1.18 ± 0.01 |
| **4** | Soya Bean-Glycerin | 68.23±0.24 | 39.79 ± 0.50 |

### Example 5

Goat milk, a raw material for a cosmetic product, was used as an herbal extraction medium as follows:
Tamarind (Tamarindus indica) was soaked in goat milk. After filtering solids from the tamarind and goat milk mixture, the goat milk infusion of tamarind was used in a cosmetic product, such as a face balsam or body milk. Active ingredients of tamarind, such as xyloglycan, were carried into the cosmetic product by the goat milk, to produce a cosmetic product that helps to improve skin elasticity, moisturize skin, heal rashes, and lighten skin color. Tamarind, which is known to contain polyphenols and antioxidants and to be an effective bio-preservative, helped to prevent deterioration of the milk.

### Example 6

In accordance with an embodiment of the present invention, multiple herbal extractions with various herbal extraction media are used, as follows:
After the calendula used in the herbal extraction of Example 3 was filtered, the solid remnant after extraction was run through another step of extraction in diluted sodium hydroxide solution and/or potassium hydroxide solution, another raw material used in soap base production as an alkaline component for a saponification reaction. With this extra step of extraction, the sunflower oil that remained on the solid calendula remnant after filtration and the remnant of calendula and other components that had not been previously extracted were recovered for extraction by the extraction medium of the sodium hydroxide and/or potassium hydroxide solution. This extra step of extraction provided a deeper level of extraction.

After the calendula was soaked in sodium or potassium hydroxide solution and filtered, the solid remnant was soaked in vinegar, another raw material of a soap base. The active materials from calendula that had not been extracted in previous steps were extracted and the solid remnant of calendula reached a suitable pH level so that it could be used in animal feed.

### Example 7

A cream was produced by heating beeswax and the oil infusion in the oil phase (mixture of oils listed in Table 9 below) to about 80 degrees Celsius. Lecithin, propolis and xanthan gum were mixed into the water phase that included the extracts in vinegar, glycerol and water. The oil phase and the water phase mixtures were then mixed under vigorous stirring to produce a cream.

**Table 9. Cream mixture**

| Cream Formulation | Parts |
|---|---|
| Manuka essential oil | 1 |
| Lavander essential oil | 0.2 |
| Sandalwood oil | 1 |
| Rosewood oil | 1 |
| Coconut oil | 3 |
| Calendula infusion in olive oil | 2 |
| Sesame oil | 2 |
| Apricot kernel oil | 2.5 |
| Grapeseed oil | 2.5 |
| Rosehip oil | 1 |
| Propolis | 0.5 |
| Beeswax | 3 |
| Lecithin | 3 |
| Black elderberry water extract | 29.3 |
| Rosehip water extract | 30 |
| Mallow glycerin extract | 5 |
| Helichrysum vinegar decoction | 10 |
| Xanthan gum | 3 |

The foregoing examples have been provided merely for the purpose of explanation and are in no way to be construed as limiting of the present invention. While the present invention has been disclosed with reference to embodiments, the words used herein are intended to be words of description and illustration, rather than words of limitation. While the present invention has been described with reference to particular materials and embodiments, the present invention is not intended to be limited to the particulars disclosed herein. For example, various types of vinegar, with various concentrations of acetic acid, and various types of vinegar infusions with various concentrations of herbs, as disclosed above, can be alternatives which may be combined in various applicable and functioning combinations within the scope of the present invention. Rather, the present invention extends to all functionally equivalent structures, materials, and uses, such as are within the scope of the appended claims. Changes may be made, within the purview of the appended claims, as presently stated and as may be amended, without departing from the scope and spirit of the present invention. All terms used in this disclosure should be interpreted in the broadest possible manner consistent with the context.

## Claims

1. An herbal extraction method for natural products, the method comprising:
• extracting soluble components of a first herb with a first extraction medium to provide a first herbal extract infusion, wherein the first extraction medium is a first raw material for producing a cosmetic product;
• extracting soluble components of a second herb with a second extraction medium to provide a second herbal extract infusion, wherein the second extraction medium is a second raw material for producing the cosmetic product; and
• mixing the first herbal extract infusion with the second herbal extract infusion to produce the cosmetic product.

2. The method according to claim 1, wherein the first extraction medium is selected from the group consisting of natural vinegar, turpentine, oil, fat, milk, sodium hydroxide, potassium hydroxide, glycerin, water, and a combination thereof.

3. The method according to claim 2, wherein the natural vinegar can be selected from the group consisting of apple vinegar, rice vinegar, wine vinegar, cider vinegar, lemon vinegar, sugar beet vinegar, sugar cane vinegar, blackberry vinegar, raspberry vinegar, and any combination thereof.

4. The method according to claims 2 or 3, wherein natural vinegar has acetic acid concentration between 2 wt% and 30 wt%.

5. The method according to claim 2, wherein oil can be selected from the group consisting of olive oil, sunflower seed oil, corn oil, coconut oil, palm oil, palm kernel oil, castor oil, and any combination thereof.

6. A method according to claim 1, wherein the second extraction medium is a second saponification reactant for producing the soap base composition.

7. The method according to claim 6,wherein the second extraction medium is one of a sodium hydroxide, a potassium hydroxide, and any combination thereof.

8. The method according to any one of the claims 1 to 7, wherein saponification reaction takes place at a temperature between 70 degrees Celsius and 99 degrees Celsius .

9. The method according to any one of the claims 1 to 8, wherein the first extraction medium is vinegar and the herb solvated in vinegar is selected from a group consisting of Calendula (Calendula officinalis), Nettle (Urtica dioica), Green tea (Camellia sinensis), Horsetail (Equisetum arvense), Lavender (lavendula officinalis), Rosemary (Rosmarinus officinalis), Sage (Salvia officinalis), Melissa (Melissa officinalis), Olive leaves (Olea europaea), Thyme (Thymus vulgaris), Carob (Ceratonia siliqua), Dandelion (Taraxacum officinale), Cinnamon (cinnamomum zeylanicum), Cloves (sysygium aromaticum), Redclover (Trifolium pratense), Motherwort (Leonurus cardiaca), Burdock (Arctium lappa), Tarragon(Artemisia dracunculus), Basil (Ocimum basilicum), Mint (Mentha piperita), Dill (Anethum graveolens), Chamomile flowers (Matricaria chamomilla), Ginseng (Panax ginseng), Alfalfa (Medicago sativa), Citrus peels (citrus), Yarrow (Achillea millefolium), Licorice (Glycyrrhiza glabra), Digitalis (Digitalis purpurea), Gentian root (Gentiana Lutea), Yellow dock (Rumex crispus), Huang Qi (Astragalus membranaceus), Blue cocosh (Caulophyllum thalictroides),Wild yam (Dioscorea villosa), Pokeroot(Phytolacca americana), Senega root (Polygala senega), Comfrey (Symphytum officinale), Mullein (Verbascum Thapsus), Cayenne (Capsicum), Artichoke (Cynara scolymus),Male fern (Dryopteris filix-mas), Meadowsweet (Filipendula ulmaria), Wintergreen (Gaultheria Procumbens), Willow bark (Salix),Goldenrod (Solidago virgaurea), Skunk cabbage (symplocarpus Foetidus), Feverfew (Tanacetum parthenium), Thuja (Thuja occidentalis),Linden (Tilia), Coltsfoot (Tussilado farfara),Valerian (Valeriana officinalis),Vervain (Verbena officinalis),Periwinkle (Vinca major), Violet (Viola Odorata), Pansy (Viola tricolor),Corn (Zea mays L.),Ginger (Zingiber officinale), andany combination thereof.

10. The method according to any one of the claims 1 to 8, wherein the first extraction medium is turpentine and the herb solvated in turpentine is selected from a group consisting of Rosemary (Rosmarinus officinalis), Black cumin (Nigella sativa), Citrus peel (citrus), Sunflower (Helianthus annuus), Bayberry (Myrica Cerifera), Cloves (sysygium aromaticum), Tarragon (artemisia dracunculus), Vanilla (Vanilla planifolia), Cinnamon (cinnamomum zeylanicum), Borage (Borago officinalis), Burdock (Arctium lappa), Cedar (Cedrus atlantica), Mint (Mentha piperita), Comfrey (Symphytum officinale), Echinacea (Echinacea angustifolia), Elderberry (Sambucus nigra), Nettle (Urtica dioica), Eucalyptus (Eucalyptus globulus), Ginkgo (Ginkgo biloba), Horehound(Marrubium vulgare), Sage (Salvia officinalis), Marshmallow (althaea officinalis), Rhubarb (Rheum officinale), St John's wort (Hypericum perforatum), Thyme (Thymus vulgaris), Witch hazel (Hamamelis virginiana), Yarrow (Achillea millefolium), Acacia (Acacia Senegal), Tragacanth (Astragalus gummifer), Irish moss (Chondrus crispus), Cluster beans (Cyamopsis tetragonoloba), Quince leaves(Cydonia Oblonga), Psyllium (plantago ovata), Fenugreek (Trigonella Foenum-Graecum), Coltsfoot (Tussilado farfara), Slippery elm (ulmus rubra), Shallaki (Boswellia serrata), Camphor laurel (Cinnamomum Camphora), Myrrh (Commiphora molmol), Dragon's blood (Daemonorops draco), Grindelia (Grindelia), Lignum vitae (Guaiacum officinale), Dandelion (Taraxacum officinale), Tansy (Tanacetum vulgare), Rue (Ruta graveolens), Bogbean (Menyanthes Trifoliata), Goldenseal (Hydrastis Canadensis), Gentian root (Gentiana Lutea), Boneset (Eupatorium perfoliatum), Centaury (Erythraea centaurium), Barberry (Berberis vulgaris), and any combination thereof.

11. The method according to any one of the claims 1 to 8, wherein, the first extraction medium is plant oil and the herb solvated in plant oil is selected from a group consisting of Calendula (Calendula officinalis), Lavender (Lavendula officinalis), Sage (Rosmarinus officinalis), Yarrow (Achillea millefolium), Burdock (Arctium lappa), Licorice (Glycyrrhiza glabra), Ginseng (Panax ginseng), Dandelion (Taraxacum officinalis), Digitalis (Digitalis purpurea), Gentian root (Gentiana Lutea), Yellow dock (Rumex crispus), Huang Qi (Astragalus membranaceus), Blue cocosh (Caulophyllum thalictroides), Wild yam (Dioscorea villosa), Pokeroot (phytolacca americana), Senega root (Polygala senega), Comfrey (Symphytum officinale), Mullein (Verbascum Thapsus), Marshmallow (althaea officinalis), Arnica (Arnica Montana), Eucalyptus (Eucalyptus globulus), Goldenseal (Hydrastis Canadensis), Elderberry (Sambucus nigra), Chickweed (Stellaria media), Thyme (Thymus vulgaris), Slippery elm (Ulmus rubra), Malva (Malva sylvestris), Cranesbill (Geranium maculatum), Tea tree(Melaleuca), and any combination thereof.

12. The method according to any one of the claims 1 to 8,wherein the first extraction medium is milk and the herb solvated in milk is selected from a group consisting of Tamarind (Tamarindus indica), Olive leaves (Olea europaea), Cloves (sysygium aromaticum), Cinnamon (cinnamomum zeylanicum), Thyme (Thymus vulgaris), Quince leaves (Cydonia Oblonga),Yarrow (Achillea millefolium),Horse chestnut (Aesculus hippocastanum),Buchu (Agathosma betulina),Agrimony (Agrimonia eupatoria),Parsley piert (Alchemilla arvensis),Marshmallow (Althaea officinalis),Dill (Anethum graveolens), Angelica (Angelica archangelica),Celery (apium graveolens),Wormwood(Artemisia absinthium),Oat (Avena Sativa), Black horehound (Ballota nigra), Calendula (Calendula officinalis), Shepherd's-purse (Capsella bursa-pastoris), Cayenne pepper (Capsicum annuum),Cummin (Carum Carvi),Iceland moss (Cetraria islandica),Irish moss (Chondrus crispus), Hawthorn (Crataegus laevigata),Broom (Cytisus scoparius),Carrot (Daucus carota),Sundew (Drosera rotundifolia),Couch grass (Elymus repens), Ma huang (Ephedra sinica),California poppy (Eschscholtzia Californica),Eucalyptus (Eucalyptus globulus),Boneset (Eupatorium perfoliatum),Eyebright (Euphrasia),Meadowsweet (Filipendula ulmaria), Fennel (Foeniculum vulgare), Fumitory (Fumaria officinalis),Goat's rue (Galega officinalis),Cleavers (Galium aparine),Cranesbill (Geranium maculatum),Ginkgo (Ginkgo biloba),Grindelia (Grindelia),Mouse-ear hawkweed (Hieracium pilosella),Hop (Humulus lupulus),St John's wort (Hypericum perforatum),Hyssop (Hyssopus Officinalis), Juniper (Juniperus Communis), Wild lettuce (Lactuca Virosa), Lavender (Lavendula officinalis), Motherwort (Leonurus cardiaca),Flax (Linum usitatissimum), Bugleweed (Lycopus europaeus),Malva (Malva sylvestris),Horehound (Marrubium vulgare),Chamomile flowers (Matricaria chamomilla),Melissa (Melissa officinalis),Mint (Mentha piperita),Pennyroyal (Mentha pulegium),Wintergreen (Mitchella repens), Catnip (Nepeta cataria),Passion flower (Passiflora incarnata),Parsley (Petroselinum Crispum),Boldo (Peumus boldus), Pokeroot (Phytolacca americana), Anise (Pimpinella anisum),Plantain (Plantago major), Senega root (Polygala senega),Pulsatilla (Pulsatilla vulgaris),Rosemary (Rosmarinus officinalis), Raspberry (Rubus idaeus),Rue (Ruta graveolens),Sage (Salvia Officinalis),Elderberry (Sambucus nigra),Figwort (Scrophularia nodosa),Skullcap (Scutellaria Lateriflora),Senna (Senna), Saw palmetto (Serenoa repens),Goldenrod (Solidago virgaurea), Chickweed (Stellaria media), andany combination thereof.

13. The method according to any one of the claims 1 to 8, wherein the first extraction medium is glycerol and the herb solvated in glycerol is selected from a group consisting of Rosmarinic acid containing plants, Agastache rugosa (Korean mint), Anethum graveolens (dill), Artemisia dracunculus (Tarragon), Borago officinalis Borage, Collinsonia canadensis (Canada Horsebalm), Glechoma hederacea (ground-ivy), Hyssopus officinalis (hyssop), Lavandula angustifolia (lavander), Lavandula x intermedia (lavandin), Lavandula latifolia (Portuguese lavender), Lycopus europaeus (gypsywort), Lycopus virginicus, Melissa officinalis (lemon balm), Mentha pulegium pennyroyal, Mentha spicata (Spearmint), Mentha longifolia (Horse Mint), Mentha arvensis var. Piperascens (corn mint), Mentha aquatica (water mint), Mentha x piperita (Peppermint), Mentha x rotundifolia, Momordica charantia (bitter melon), Monarda fistulosa Wild bergamot, Monarda Didyma, Nepeta cataria (catnip), Ocimum tenuiflorum (holy basil), Ocimum basilicum (basil), Origanum vulgare (Oregano), Origanum majorana (Marjoram), Perilla frutescens, Petroselinum crispum, Prunella vulgaris common self-heal, Rosmarinus officinalis
(rosemary), Salvia officinalis (Sage tea), Salvia sclarea (clary sage), Satureja hortensis (Summer savory), Satureja montana (Winter savory), Scrophularia scorodonia (figworts), Stachys officinalis (hedgenettle), Symphytum officinale
(common comfrey), Teucrium scorodonia(wood sage), Thymus vulgaris (German thyme), Thymus serpyllum (wild thyme), Vaccinium corymbosum (high blueberry), Carnosic acid containing plants: Hyptis dilatata, Lepechinia meyenii, Lepechinia hastata, Ocimum tenuiflorum (holy basil), Pulicaria salviaefolia, Rosmarinus officinalis (rosemary), Salvia munzii (Munz's sage), Salvia canariensis (Canary Island sage), Salvia apiana (white sage), Salvia mellifera (black sage), Salvia willeana (Troodos Sage),Salvia officinalis (Sage tea), Salvia tomentosa, Salvia columbariae (chia sage), GENISTEIN containing plants: Adenocarpus foliolosus, Adenocarpus decorticans, Albizia procera, Amphicarpaea edgworthii, Amphicarpaea bracteata hog peanut, Apios americana, Argyrocytisus battandieri, Baptisia sp, Baptisia tinctoria Wild Indigo, Bowdichia nitida, Cajanus cajan Pigeonpea, Cajanus scarabaeoides, Calicotome villosa, Calicotome spinosa, Calopogonium caeruleum, Camptosema sp, Canavalia eurycarpa, Canavalia rosea, Canavalia ensiformis Jack Bean, Canavalia virosa, Canavalia gladiata Kachang polong; Carabanz; Kachang parang (Chopper bean), Canavalia galeata, Canavalia sericea, Centrosema schiedianu, Centrosema pascuorum, Centrosema sagittatum, Centrosema plumieri, Chamaecytisus supinus, Chamaecytisus hirsutus, Chamaecytisus smyrnaeus, Chamaecytisus eriocarpus, Chamaecytisus ratisbonensis, Chamaecytisus albus, Chamaespartium tridentatum, Chamaespartium sagittale, Chronanthus biflorus, Cicer arietinum Chickpea; Garbanzo, Clitoria ternateaKembang teling; Mazeryon Hindi; Menteling; Cwetabhanda , Clitoria falcata, Cologana broussonetii, Crotalaria juncea Sunhemp, Cytisus multiflorus, Cytisus tribracteolatus, Cytisus ingramii, Cytisus scoparius Scotch Broom, Cytisus striatus, Cytisus ardoini Desmodium gangeticum Serengan; Vidarigandha; Lepantan; Akar katuh; Kemani bali; Meringan Dioclea glycinoides Dipogon lignosus, Dolichos biflorus Kulattha, Dunbaria villosa Wild Yam, Echinospartium horridum, Erinacea anthyllis, Eriosema nutans, Eriosema glomeratum Mampong, Eriosema psoraleoides, Erythrina crista-galli Cockspur Coral Tree, Erythrina sp, Flemingia strobilifera, Galactia jussiaeana, Genista patula, Genista germanica, Genista sp genet, Genista ovata, Genista clavata, Genista tinctoria, Genista rumelica, Genista Lydia, Glycine max soybean, Glycine wightii, Glycyrrhiza glabra licorizei, Hardenbergia violacea, Kennedia nigricans (Black Kennedia, Kennedia rubicunda dusky coral pea, Kennedia coccinea (coral vine), Kennedia procurrens, Laburnum alpinum Scotch laburnum, Laburnum anagyroides golden rain, Lembotropis emerifolia, Lens culinaris lentil, Lespedeza cyrtobotrya bush clovers, Lupinus albus white lupin, Lupinus luteus European yellow lupine, Lupinus perennis wild perennial lupine,, Lygos raetum, Lygos monosperma, Maackia amurensis, Macroptylium bracteatum, Macroptylium martii, Macroptylium atropurpureum, Macroptylium lathyroides, Macrotyloma axillare, Macrotyloma uniflora, Medicago sativa, Moghania macrophylla, Mucuna deeringiana, Mucuna pruriens, Neonotonia wightii, Ononis spinosa, Pericopsis laxiflora, Phaseolus lunatus, Phaseolus vulgaris, Phaseolus spp, Phaseolus lunatus, Phaseolus coccineus, Piptanthus nepalensis, Piptanthus nanus, Pisum sativum, Podocarpus spicatus, Prunus nipponica, Prunus avium, Prunus aequinoctialis, Prunus verecunda, Prunus maximowiczii, Prunus cerasus, Prunus spp, Prunus mahaleb, Pseudeminia comosa, Pseudoeriosema borianii, Pseudovigna argentea, Psoralea corylifoliaPterocarpus angolensisPueraria pseudohirsutaPueraria phaseoloidesPueraria montana, Rhynchosia densiflora, Rhynchosia pyramidalis, Rhynchosia cabibaea, Rhynchosia phaseoloides, Rhynchosia minima, Rhynchosia acuminatifolia, Rhynchosia volubilis, Rhynchosia hirsuta, Sophora japonica, Sophora subprostrata, Spartium junceum, Stauracanthus genistoides, Stauracanthus boivinii, Stellaria media, Stizolobium deeringianum, Strongylodon macrobotrys, Teline sp, Teyleria koordersii, Thermopsis sp, Thermopsis fabacea, Trifolium subterraneum, Trifolium hybridum, Trifolium pratense, Trifolium sp, Trifolium brachycalycinum, Trifolium repens, Trifolium alexandrinum, Trigonella foenum-graecum, Ulex sp, Ulex micranthus, Vandasina retusa, Vicia faba, Vigna angularis, Vigna unguiculata, Vigna subterranea, Vigna radiata, GENISTIN containing plants:Genista tinctoria dyer's broom, Glycine max soybean, Prunus cerasus sour cherry, Pueraria montana kudzu, Trifolium pratense red clover, Ulex europaeus common gorse, DAIDZEIN containing plants:Cicer arietinum Chickpea, Genista tinctoria Dyer's Broom, Glycine max Soybean, Lens culinaris Lentil, Medicago sativa alfalfa, Phaseolus lunatus butter bean, Phaseolus vulgaris black bean, Phaseolus coccineus scarlet runner bean, Pisum sativum pea, Psoralea corylifolia babchi, Pueraria pseudohirsuta chinese kudzu, Pueraria montana kudzu, Sophora subprostrata Shan Dou Gen, Trifolium pratense Peavine Clover, Vicia faba Faba Bean, Vigna radiata Mungbean, Vigna angularis Adzuki Bean, Vigna unguiculata Asparagus Bean, DAIDZIN containing plants:Glycine max Soybean, Pueraria montana kudzu, Pueraria pseudohirsuta chinese kudzu, Trifolium pratense Peavine Clover, and any combination thereof.
